(19) Europäisches Patentamt
European Patent Office
Office européen des brevets

(11) Publication number: 0 369 388 B1

(12) # EUROPEAN PATENT SPECIFICATION

(45) Date of publication of patent specification: **19.05.93**

(51) Int. Cl.[5]: **C07D 219/10**, A61K 31/435, C07D 219/12

(21) Application number: **89121037.9**

(22) Date of filing: **14.11.89**

The file contains technical information submitted after the application was filed and not included in this specification

(54) **Hydroxy-1,2,3,4-tetrahydroaminoacridines, a process for their preparation and their use as medicaments.**

(30) Priority: **16.11.88 US 271788**

(43) Date of publication of application: **23.05.90 Bulletin 90/21**

(45) Publication of the grant of the patent: **19.05.93 Bulletin 93/20**

(84) Designated Contracting States: **AT BE CH DE ES FR GB GR IT LI LU NL SE**

(56) References cited:
**EP-A- 0 179 383**
**EP-A- 0 282 959**

(73) Proprietor: **HOECHST-ROUSSEL PHARMACEUTICALS INCORPORATED**
**Route 202-206 North**
**Somerville New Jersey 08876(US)**

(72) Inventor: **Shutske, Gregory Michael**
**47 Cedarbrook Drive**
**Somerset, N.J. 08873(US)**
Inventor: **Tomer, John Dick IV**
**236 Allem Lane**
**Perkasie, PA 18944(US)**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80 (DE)**

EP 0 369 388 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to compounds having the formula

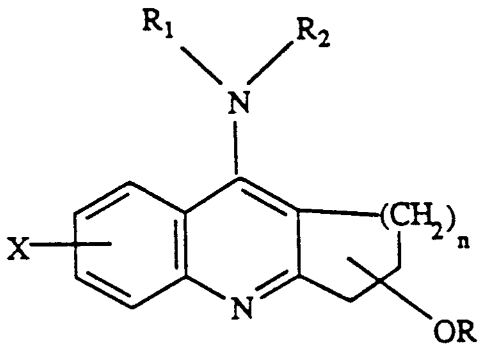

(I)

where

| | |
|---|---|
| n | is 2; |
| X | is hydrogen or halogen |
| R | is hydrogen, benzyl or alkanoyl of 2-12 carbons, with the proviso that the group -OR may not be on the 1-position of the three ring system, or if R is hydrogen, the group -OR may not be on the 4-position of the three ring system; and |
| $R_1$ and $R_2$ | are each independently hydrogen, loweralkyl or arylloweralkyl, but both may not be arylloweralkyl simultaneously, or alternatively $R_1$ and $R_2$ taken together may constitute the group |

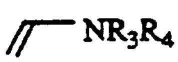

where $R_3$ and $R_4$ are each independently loweralkyl;

which are useful for the treatment of various memory dysfunctions characterized by decreased cholinergic function, such as Alzheimer's disease.

9-Amino-1,2,3,4-tetrahydro-acridines and related compounds wherein the benzene ring and optionally, the amino group is substituted, a process for their preparation and their use as medicaments are disclosed in EP-A-282 959.

9-Amino-1,2,3,4-tetrahydroacridin-1-ol and related compounds, whose benzene ring and amino group is optionally substituted, a process for their preparation and their use as medicaments are known from EP-A-179 383.

EP-A-268 871 discloses 9-amino-quinoline derivatives encompassing among others 9-amino-1,2,3,4-tetrahydroacridines where optionally one of the positions 1 to 4 is substituted by lower alkyl.

Moreover WO 88/02256 discloses 9-monoamine acridine derivatives, where optionally positions 1, 2 and 3 or 2, 3 and 4 are substituted by hydrogen and position 4 or 1 is substituted by a hydroxy group and their use in the treatment of the centralnervous system and peripheral nervous system cholinergic deficit states.

Subgeneric to compounds I depicted above are compounds of Formula Ia and Formula Ib depicted below.

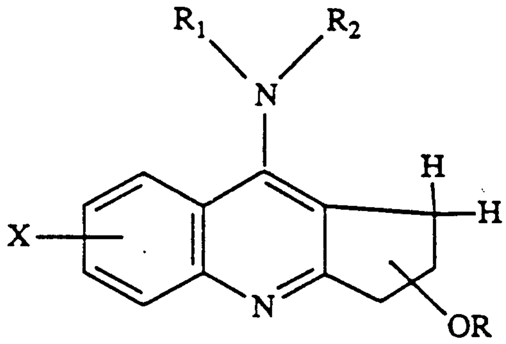

(Ia)

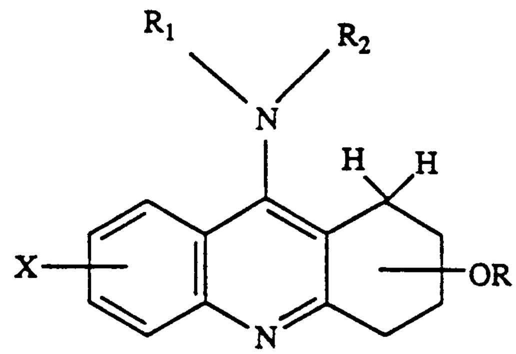

(Ib)

Preferred compounds of the formula I are those where the group OR is in 2 – position.

In particular preferred are compounds of the formula I, wherein $R_1$, $R_2$ and X are hydrogen.

Furthermore preferred are compounds of the formula I, wherein R is hydrogen or $C_2 - C_6$ – alkanoyl.

Throughout the specification and the appended claims, a given chemical formula or name shall encompass all stereo, geometrical and optical isomers thereof where such isomers exist, as well as pharmaceutically acceptable acid addition salts thereof and solvates thereof such as for instance hydrates.

The following definitions shall apply throught the specification and the appended claims.

Unless otherwise stated or indicated, the term loweralkyl denotes a straight or branched alkyl group having from 1 to 6 carbon atoms. Examples of said loweralkyl include methyl, ethyl, n – propyl, iso – butyl, pentyl and hexyl.

Unless otherwise stated or indicated, the term cycloalkyl denotes an alicyclic hydrocarbon group containing 3 to 7 carbon atoms. Examples of said cycloalkyl include cyclopropyl, cyclohexyl and cycloheptyl.

Unless otherwise stated or indicated, the term halogen shall mean fluorine, chlorine, bromine or iodine.

Unless otherwise stated or indicated, the term aryl shall mean an unsubstituted phenyl group or a phenyl group substituted with 1, 2 or 3 substituents each of which being independently loweralkyl, loweralkoxy, halogen, hydroxy or trifluoromethyl.

The compounds of this invention are prepared by utilizing one or more of the steps described below.

Throughout the description of the synthetic steps, the definitions of n, X, R, $R_1$ and $R_2$ are as given above unless otherwise stated or indicated.

SCHEME 1:

Preparation of 2-ols of Formula I(c) depicted below.

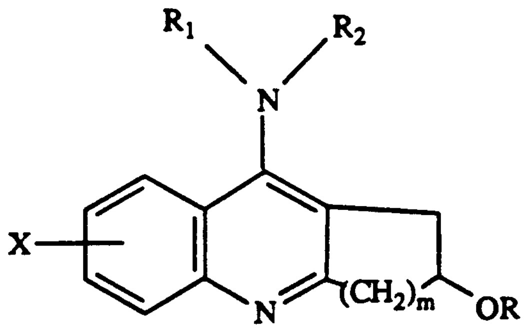

m = 1 or 2

(Ic)

STEP 1(a)

An amino nitrile compound depicted below is allowed to react with a cyclic ketone depicted below to afford a compound of Formula II.

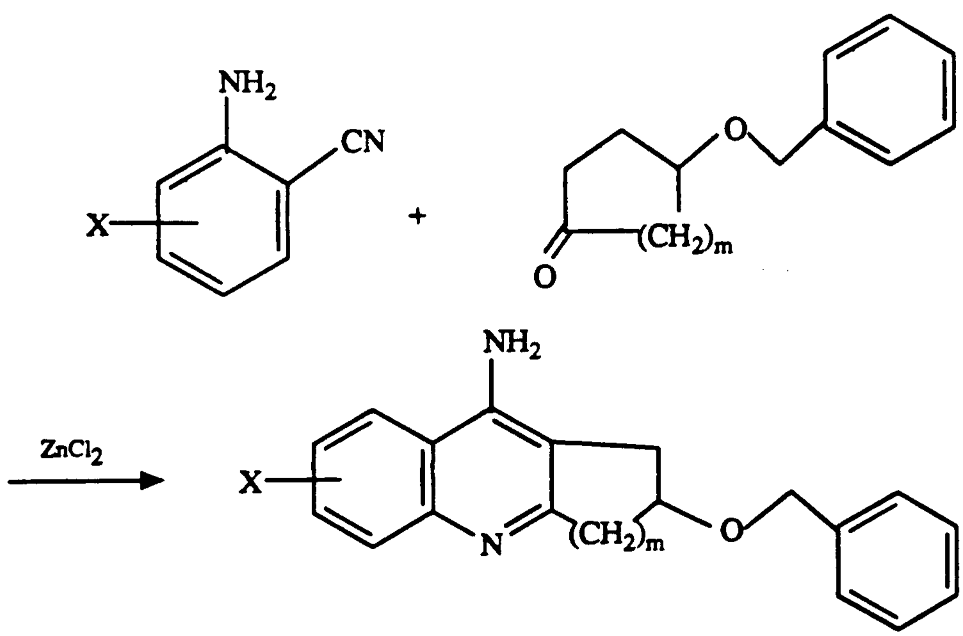


(II)

Said reaction is conducted in the presence of a Lewis acid such as zinc chloride and a suitable solvent such as nitrobenzene, toluene or 1,2-dichloroethane at a temperature of about 80° - 150° C.

STEP 1(b)

Compound II (where X is not OH) is allowed to react with a compound of the formula $R_1W$, W being Cl, Br, I or $OSO_3CH_3$ (mesyloxy) to afford a compound of Formula III. Typically, said reaction is conducted in a biphasic system comprising a suitable organic solvent such as dichloromethane, chloroform, benzene,

toluene or the like, a strongly alkaline aqueous phase such as 50% aqueous NaOH or the like, the starting compounds and a phase transfer catalyst such as tetrabutylammonium hydrogensulfate at a temperature of about 0° − 50° C.

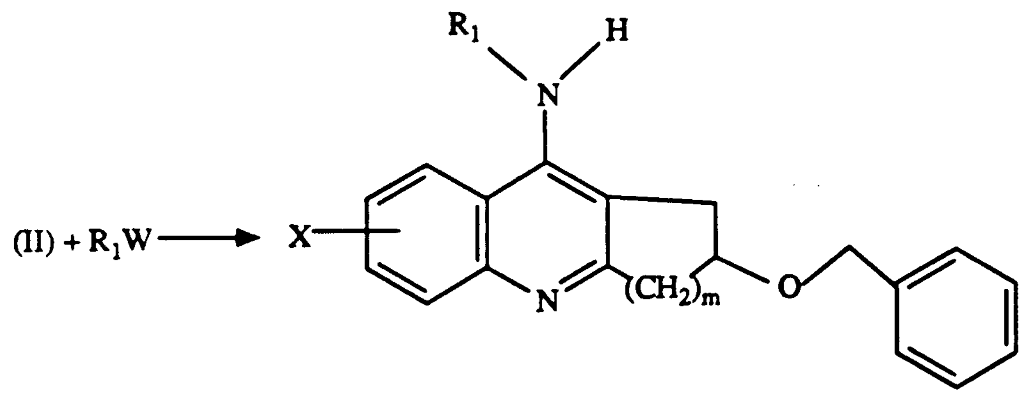

(III)

$R_1 \neq H$
$X \neq OH$

Where X is OH, the excluded compound can be prepared by subjecting a compound of Formula V where X is a loweralkoxy group (e.g. methoxy group) to a cleavage reaction conducted, for instance, with the aid of pyridine hydrochloride at a temperature of around 180° C.

STEP 1(c)

Compound III is allowed to react with a compound of the formula $R_2W$ in substantially the same manner as in STEP 1(b) to afford a compound of Formula IV.

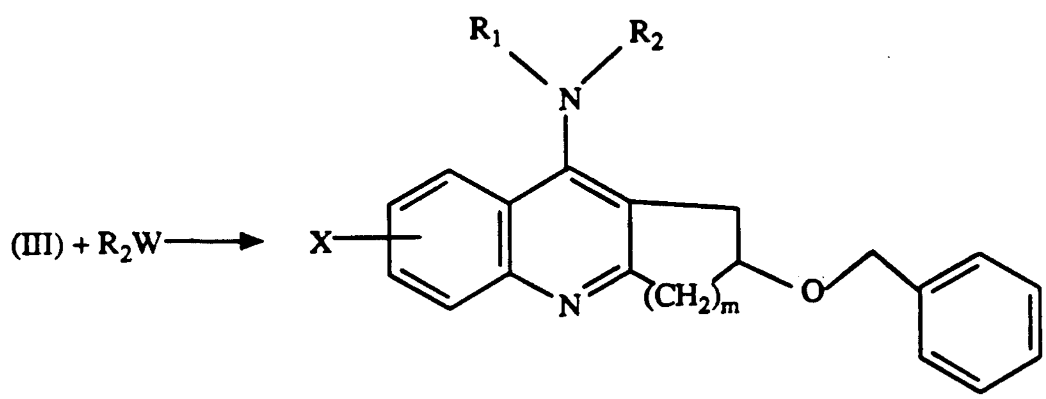

(IV)

$R_1 \neq H$, $R_2 \neq H$
$X \neq OH$

STEP 1(d)

A compound of Formula IVa (wherein $R_1$ and $R_2$ may be hydrogen as well as loweralkyl or arylloweralkyl) is subjected to hydrogenolysis in a routine manner known to the art to afford a compound of Formula V. A preferred catalyst for this reaction is paladium on carbon.

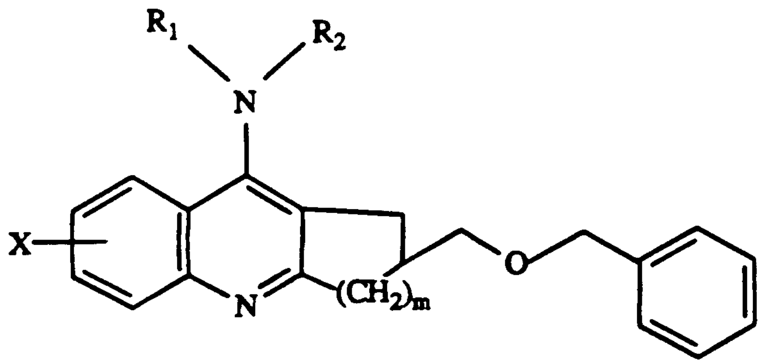

(IVa)

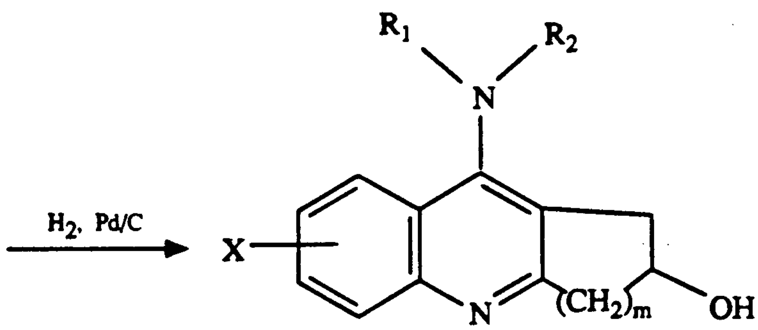


(V)

STEP 1(e)

Compound V is allowed to react with an acyl chloride of Formula VI or an ester of Formula VIa where the group $R_5$ is an alkyl group of 1 – 11 carbon atoms and the group $R_6$ is an alkyl group of one or two carbon atoms in a routine manner known to the art to afford a compound of Formula VII.

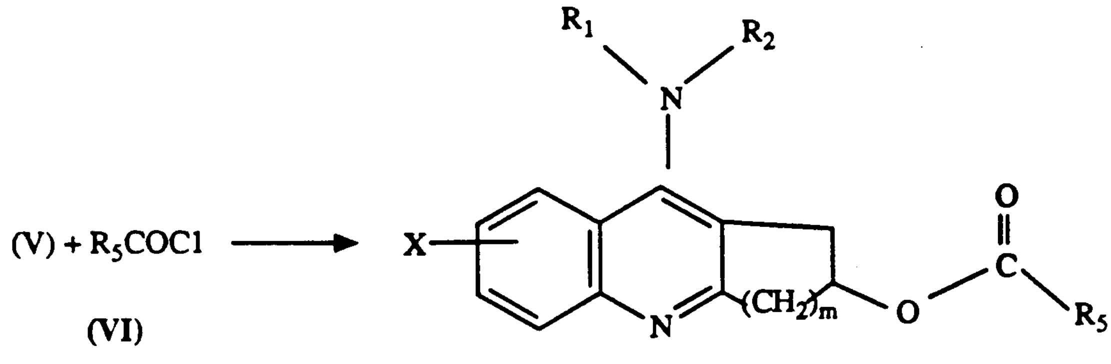

(VI)

(VII)

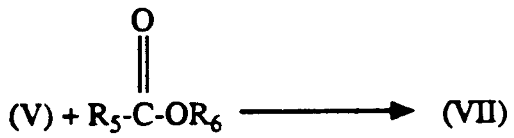

(VIa)

Step 1(f)

Compound V is allowed to react with a formamide acetal of Formula VIII where $R_3$, $R_4$ and $R_7$ are loweralkyl in a routine manner known to the art to afford a compound of Formula IX.

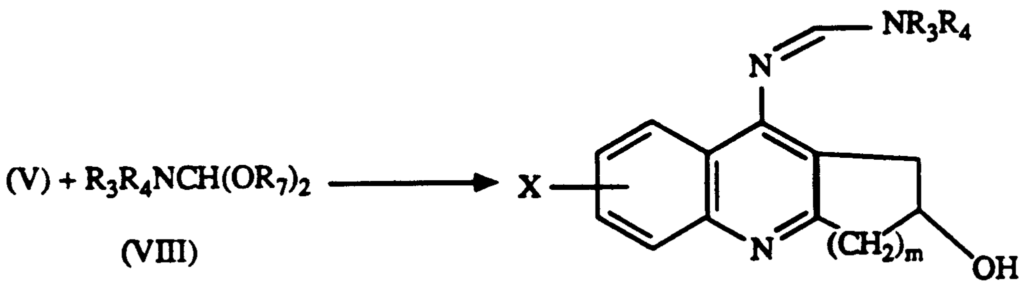

(VIII)

(IX)

SCHEME 2:

Preparation of 9 – amino – 1,2,3,4 – tetrahydroacridin – 3 – ols of Formula I(d) below.

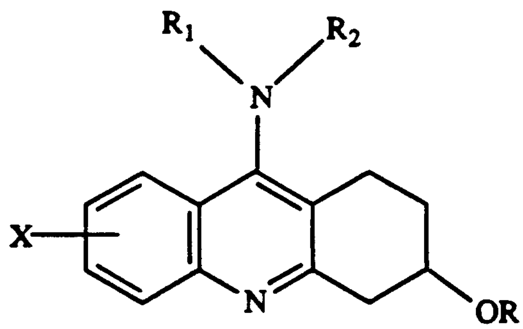

(Id)

STEP 2(a):

An amino nitrile compound depicted below is allowed to react with the cyclic ketone depicted below to afford a compound of Formula X. This reaction is conducted in substantially the same manner as in STEP 1(a).

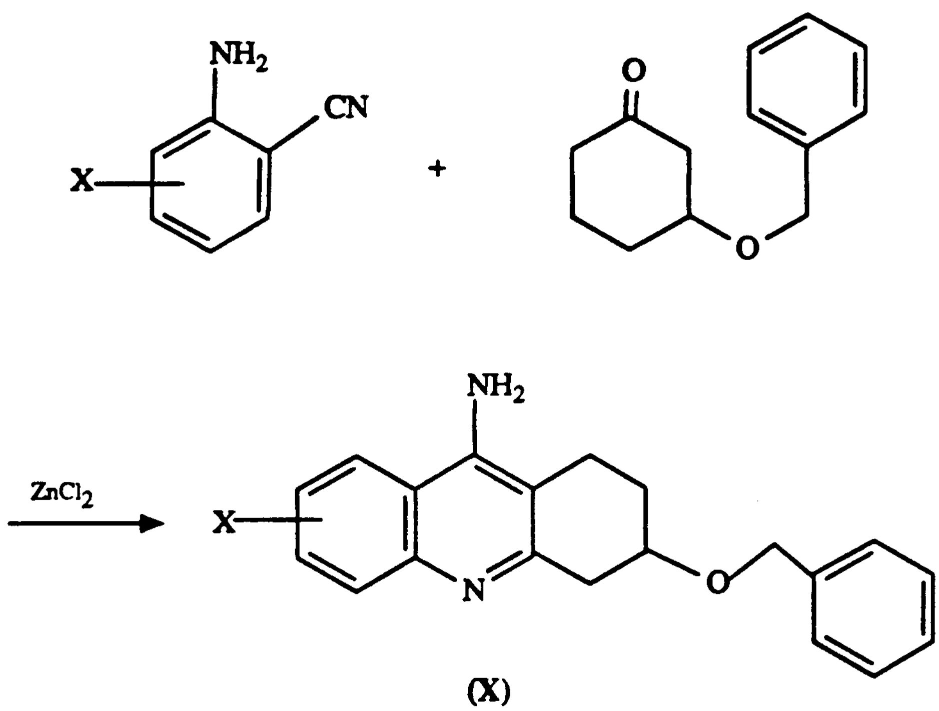


(X)

STEP 2(b):

Compound X is allowed to react with compound $R_1W$ in substantially the same manner as in STEP 1(b) to afford a compound of Formula XI.

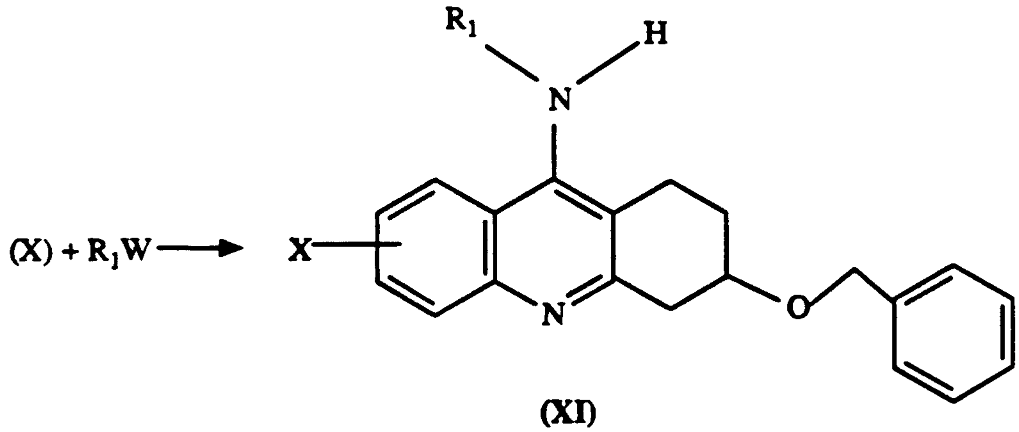


(XI)

$R_1 \neq H$, $X \neq OH$

STEP 2(c):

Compound XI is allowed to react with compound $R_2W$ in substantially the same manner as in STEP I(c) to afford a compound of Formula XII.

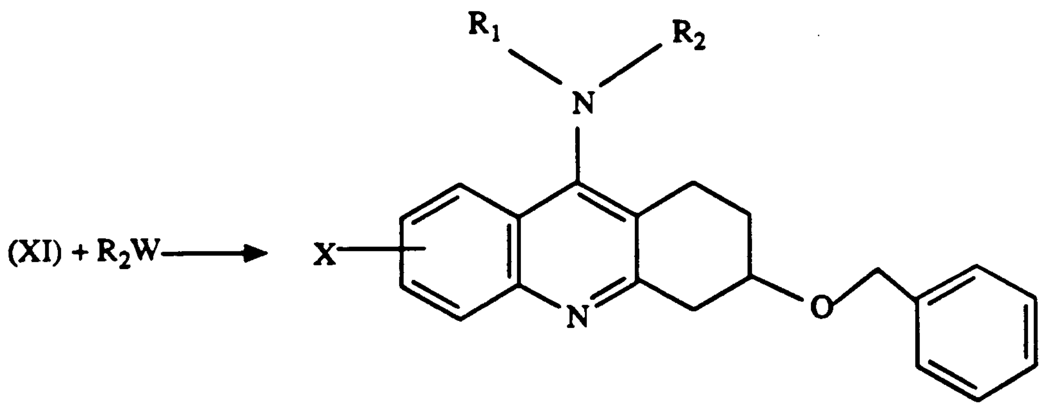


(XII)

$R_1 \neq H$, $R_2 \neq H$, $X \neq OH$

STEP 2(d):

A compound of Formula XIIa (wherein $R_1$ and $R_2$ may be hydrogen as well as loweralkyl or arylloweralkyl) is subjected to hydrogenolysis conducted in substantially the same manner as in STEP 1(d) to afford a compound of Formula XIII.

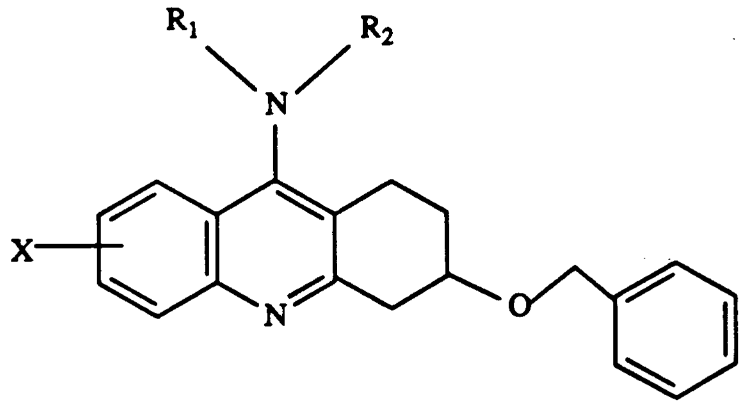

(XIIa)

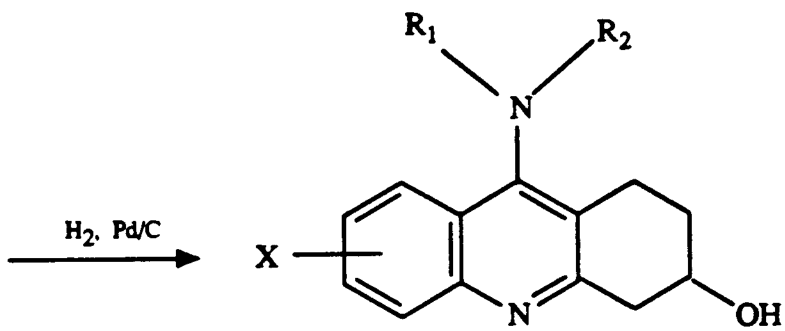


(XIII)

STEP 2(e):

Compound XIII is allowed to react with an acyl chloride $R_3COCl$ in substantially the same manner as in STEP 1(e) to afford a compound of Formula XIV.

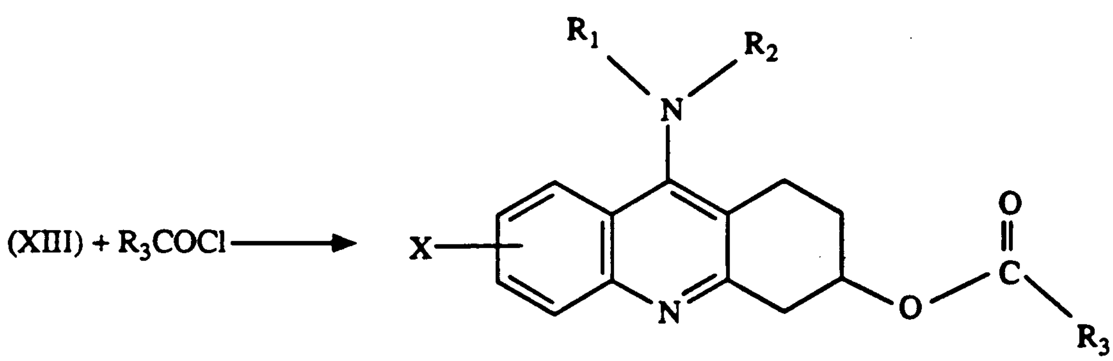


(XIV)

SCHEME 3:

Preparation of 4 – ols depicted below.

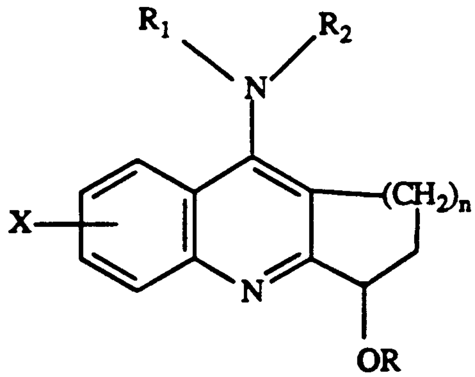

(XV)

STEP 3(a):

A compound of Formula XVI is allowed to react with acetic anhydride in a routine manner known to the art to afford a compound of Formula XVII. This reaction is known as the Polonovsky reaction.

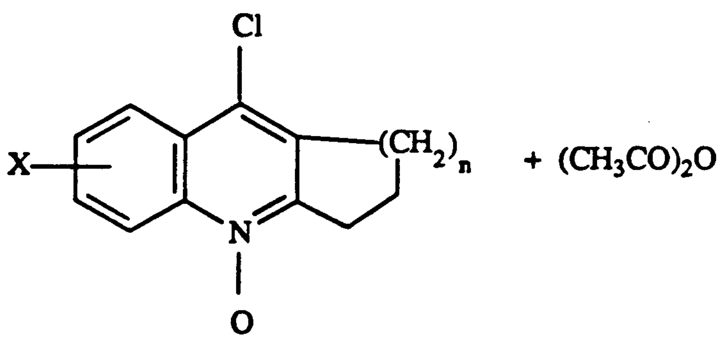


(XVI)

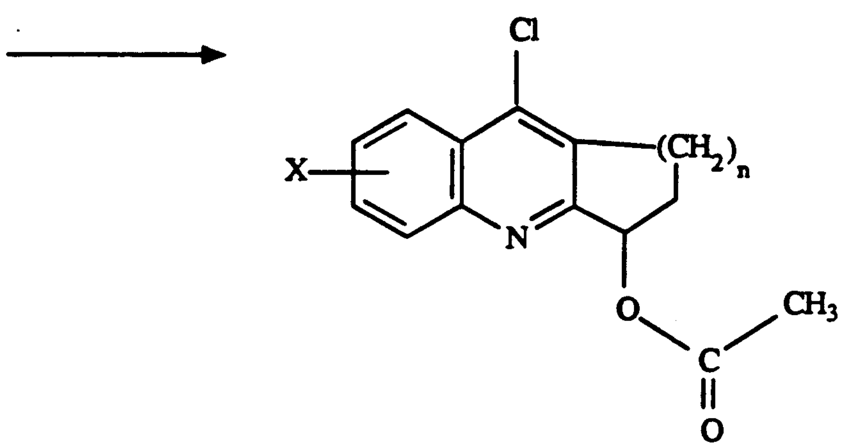

(XVII)

STEP 3(b):

Compound XVII is first hydrolized in a routine manner known to the art to obtain the corresponding alcohol and thereafter said alcohol is allowed to react with benzyl bromide in the presence of a strong base such as sodium hydride in a routine manner known in the art to afford a compound of Formula XVIII.

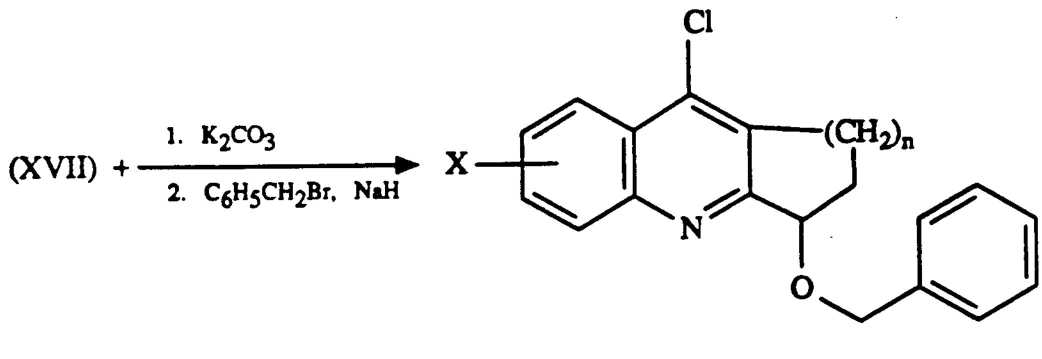


(XVIII)

STEP 3(c):

Compound XVIII is allowed to react with ammonia in a suitable medium such as phenol in a routine manner known to the art to afford a compound of Formula XIX.

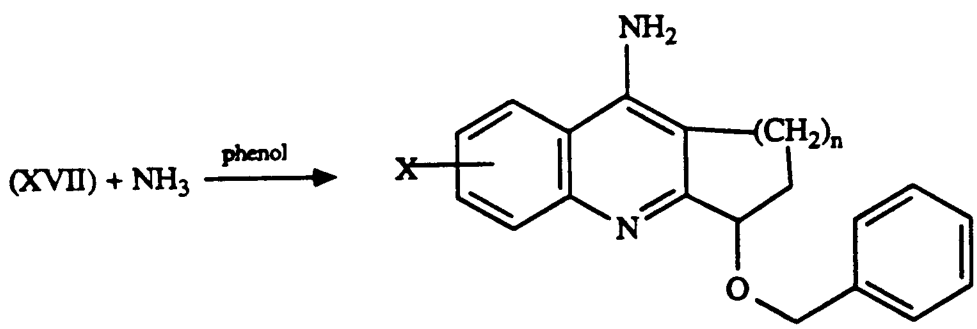


(XIX)

STEP 3(d):

Compound XIX is allowed to react with a compound $R_1W$ in substantially the same manner as in STEP 1(b) to afford a compound of Formula XX.

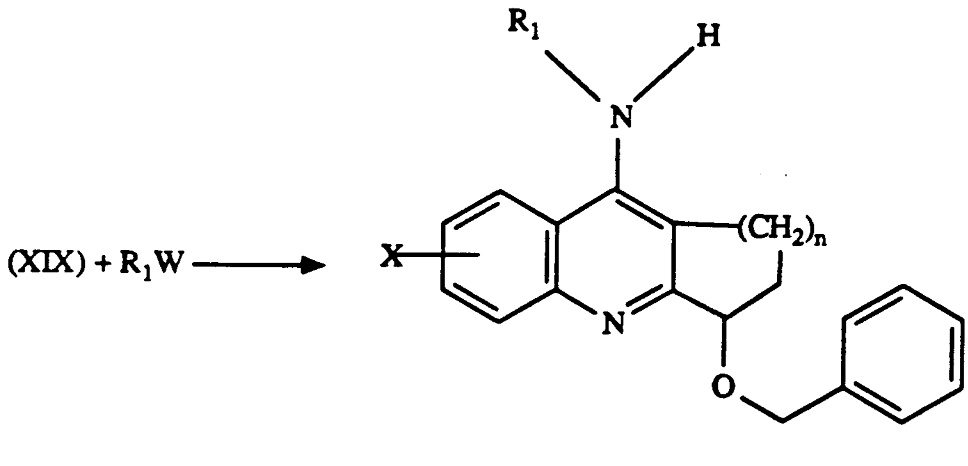


(XX)

$R_1 \neq H$, $X \neq OH$

STEP 3(e):

Compound XX is allowed to react with a compound $R_2W$ in substantially the same manner as in STEP 1(c) to afford a compound of Formula XXI.

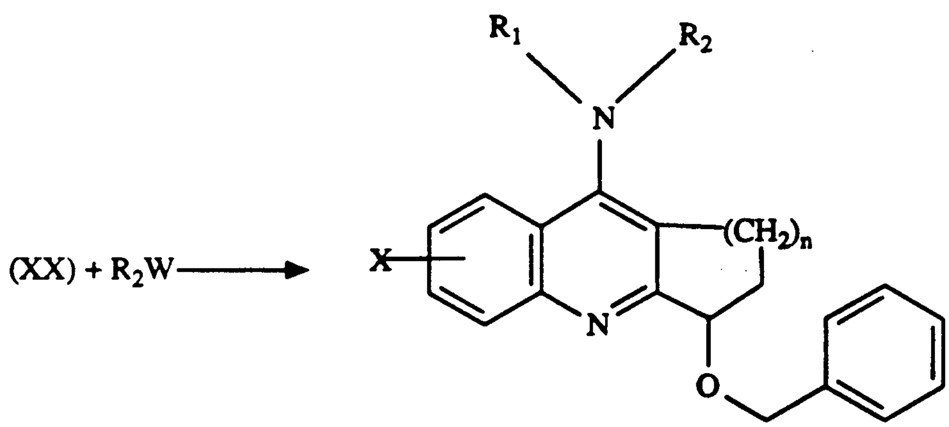


(XXI)

$R_1 \neq H$, $R_2 \neq H$, $X \neq OH$

STEP 3(f):

A compound of Formula XXIa (where $R_1$ and $R_2$ may be hydrogen as well as loweralkyl or arylloweralkyl) is subjected to a solvolysis reaction to afford a compound of Formula XXII. Typically, said solvolysis reaction is conducted in the presence of hydrogen chloride and acetic acid.

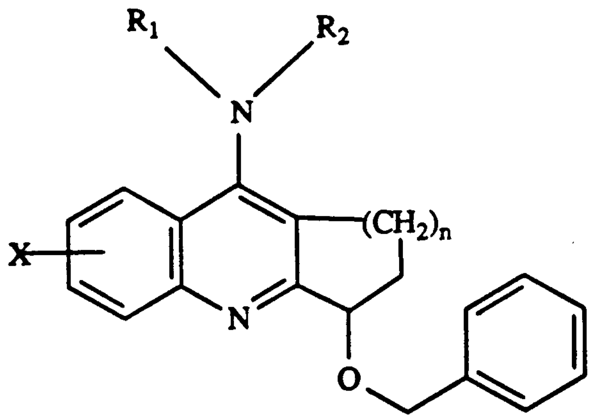

(XXIa)

$R_1 \neq H$, $R_2 \neq H$, $X \neq OH$

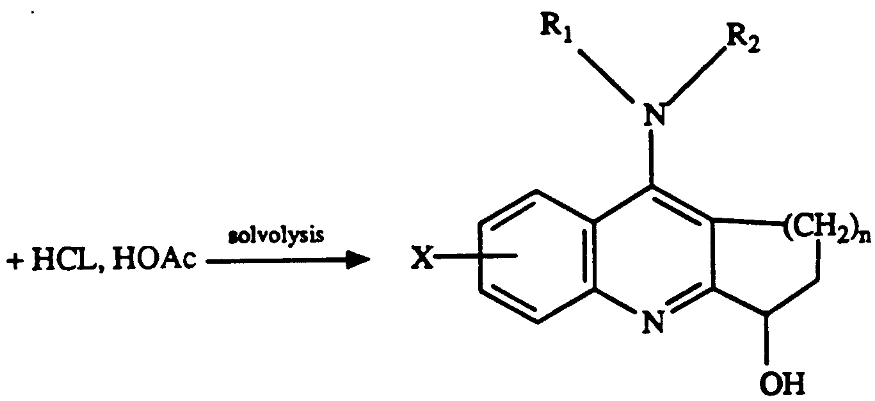


(XXII)

STEP 3(g):

Compound XXII is allowed to react with an acyl chloride $R_3COCl$ in substantially the same manner as in STEP 1(e) to afford a compound of Formula XXIII.

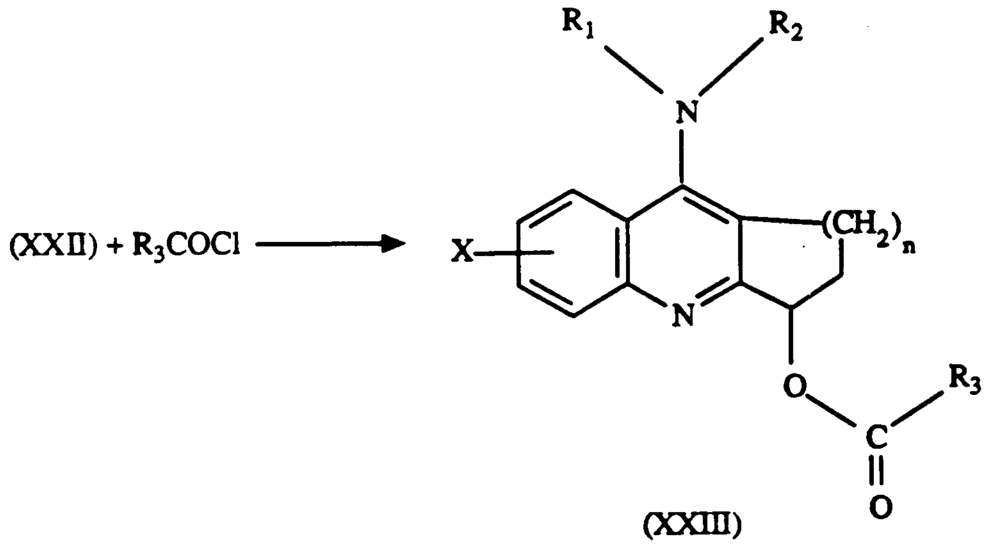


(XXIII)

The compound of Formula (I) of the present invention can be used for the treatment of various memory dysfunctions characterized by decreased cholinergic functions, such as Alzheimer's disease.

This utility can be ascertained by determining the ability of these compounds to inhibit the activity of the enzyme acetylcholinesterase and thereby increase the acetylcholine levels in the brain.

## CHOLINESTERASE INHIBITION ASSAY

The ability to inhibit acetylchlinesterase was determined by the photometric method of Ellman et al., Biochem. pharmacol. 7, 88 (1961). Results of some of the compounds of this invention are presented in Table 1 below along with those of some reference compounds.

TABLE 1 (Acetylcholinesterase Inhibition)

| Compound | $IC_{50}$ (molar conc.) |
|---|---|
| 2-Benzyloxy-1,2,3,4-tetrahydro-9-acridinamine | >1.0 x $10^{-3}$ |
| 9-Amino-1,2,3,4-tetrahydroacridin-2-ol, maleate | 5.3 x $10^{-6}$ |
| 2-Acetoxy-1,2,3,4-tetrahydro-9-acridinamine | 6.5 x $10^{-6}$ |
| 2-Hexanoyloxy-1,2,3,4-tetrahydro-9-acridinamine-fumarate | 1.9 x $10^{-5}$ |
| 2-Heptanoyloxy-1,2,3,4-tetrahydro-9-acridinamine, fumarate | 3.1 x $10^{-5}$ |
| 2-Lauroyloxy-1,2,3,4-tetrahydro-9-acridinamine | 1.6 x $10^{-4}$ |
| 2-Decanoyloxy-1,2,3,4-tetrahydro-9-acridinamine, fumarate | >1.0 x $10^{-3}$ |
| 2-Nonanoyloxy-1,2,3,4-tetrahydro-9-acridinamine, fumarate | >1.0 x $10^{-3}$ |
| 4-Benzyloxy-1,2,3,4-tetrahydro-9-acridinamine | 8.2 x $10^{-4}$ |
| 2-Butyryloxy-1,2,3,4-tetrahydro-9-acridinamine, fumarate | 4.8 x $10^{-6}$ |
| 2-Octanoyloxy-1,2,3,4-tetrahydro-9-acridinamine, fumarate | 2.3 x $10^{-5}$ |
| 2-Valeryloxy-1,2,3,4-tetrahydro-9-acridinamine, fumarate | 1.2 x $10^{-5}$ |

| | |
|---|---|
| 9-Amino-1,2,3,4-tetrahydroacridinamin-4-ol, hemifumarate | $1.9 \times 10^{-6}$ |
| 2-Hydroxy-N-(dimethylaminomethylene)-1,2,3,4-tetrahydro-9-acridinamine | $>1.0 \times 10^{-3}$ |
| (Reference Compounds) | |
| 9-Amino-1,2,3,4-tetrahydroacridine | $3.1 \times 10^{-7}$ |
| Physostigmine | $6.0 \times 10^{-9}$ |

This utility can also be ascertained by determining the ability of these compounds to restore cholinergically deficient memory in the Dark Avoidance Assay. In this assay mice are tested for their ability to remember an unpleasant stimulus for a period of 24 hours. A mouse is placed in a chamber that contains a dark compartment; a strong incadescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is blocked by an active test compound, resulting in a greater interval before re-entry into the dark compartment.

The results for an active compound are expressed as the percent of a group of animals in which the effect of scopolamine is blocked, as manifested by an increased interval between being placed in the test chamber and re-entering the dark compartment. Results of Dark Avoidance Assay for representative compounds of this invention and reference compounds are presented in Table 2.

Table 2

| Dark Avoidance Assay | | |
|---|---|---|
| Compound | Dose (mg/kg of body weight, s.c) | % of Animals with Scopolamine Induced Memory Deficit Reversal |
| 9-Amino-1,2,3,4-tetrahydroacridin-2-ol, maleate | 5.0 | 20% |
| 2-Acetoxy-1,2,3,4-tetrahydro-9-acridinamine | 5.0 | 29% |
| 2-Propionyloxy-1,2,3,4-tetrhydro-9-acridinamine, fumarate hemihydrate | 5.0 | 13% |
| 2-Hexanoyloxy-1,2,3,4-tetrahydro-9-acridinamine, fumarate | 0.63 | 25% |
| (Reference Compound) | | |
| Physostigmine | 0.31 | 20% |

Effective quantities of the compounds of the invention may be administered to a patient by any of the various methods, for example, orally as in capsule or tablets, parenterally in the form of sterile solutions or suspensions, and in some cases intravenously in the form of sterile solutions. The free final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobomic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 4% to about 70% of the weight of the unit. The amount of active compound is such compositions is such that a suitable dosage will be obtained. Preferred compositions and preparations according to the present invention are prepared so that an oral dosage unit form contains between 1.0 − 300 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweeting agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings, Thus, tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present inventions are prepared so that a parenteral dosage unit contains between 0.5 to 100 milligrams of active compound.

Examples of the compounds of this invention include:

2-Benzyloxy-1,2,3,4-tetrahydro-9-acridinamine;
9-Amino-1,2,3,4-tetrahydroacridin-2-ol;
2-Hydroxy-N-(dimethylaminomethylene)-1,2,3,4-tetrahydro-9-acridinamine;
2-Acetoxy-1,2,3,4-tetrahydro-9-acridinamine
2-Propionyloxy-1,2,3,4-tetrahydro-9-acridinamine;
2-Butyryloxy-1,2,3,4-tetrahydro-9-acridinamine;
2-Valeroyloxy-1,2,3,4-tetrahydro-9-acridinamine;
2-Hexanoyloxy-1,2,3,4-tetrahydro-9-acridinamine;
2-Heptanoyloxy-1,2,3,4-tetrahydro-9-acridinamine;
2-Octanoyloxy-1,2,3,4-tetrahydro-9-acridinamine;
2-Nonanoyloxy-1,2,3,4-tetrahydro-9-acridinamine;
2-Decanoyloxy-1,2,3,4-tetrahydro-9-acridinamine;
2-Lauroyloxy-1,2,3,4-tetrahydro-9-acridinamine;
4-Benzyloxy-1,2,3,4-tetrahydro-9-acridinamine;
9-Amino-1,2,3,4-tetrahydroacridin-4-ol;

The following examples are presented in order to illustrate this invention.

## EXAMPLE 1

### 2-Benzyloxy-1,2,3,4-tetrahydro-9-acridinamine

4-Benzyloxycyclohexanone[1] (14.86 g) and anthranilonitrile (8.90 g) were dissolved in 100 ml of nitrobenzene to which was then added freshly fused $ZnCl_2$ (0.9 g). The reaction mixture was warmed at 60° for 90 minutes and then the temperature was raised to 120° and maintained at this temperature for three hours. At the end of this time, 200 ml of anhydrous $Et_2O$ was added and the mixture was allowed to remain undisturbed overnight. The granular precipitate obtained in this manner was then filtered off, washed well with $Et_2O$, and distributed between aqueous $NH_3$ and 2-butanone. The organic phase was dried and concentrated to give a solid that was triturated well with $Et_2O$ to give 17.19 g of product, mp 188-190°.

[1]J. Chem. Soc., Chem. Commun. **1984**, 721.

Material of analytical purity was obtained by recrystallization from MeOH/$H_2O$, mp 190 - 191°.

| ANALYSIS: | | | |
|---|---|---|---|
| Calculated for $C_{20}H_{20}N_2O$:<br>Found: | 78.92%C<br>78.86%C | 6.62%H<br>6.64%H | 9.21%N<br>9.24%N |

EXAMPLE 2

9 - Amino - 1,2,3,4 - tetrahydroacridin - 2 - ol, maleate

2 - Benzyloxy - 1,2,3,4 - tetrahydro - 9 - acridinamine (18.75 g) was hydrogenated with 10% palladium/carbon in a solution prepared from absolute ethanol (1 liter) and concentrated HCl (56.83ml) at 60 psi (4.2 bar) overnight.

The reaction mixture was filtered through celite, concentrated, dissolved in deionized water (110 ml) and then 10% NaOH (55 ml) was added dropwise while the mixture was stirred in an ice bath. The resulting solid was filtered, washed with deionized water and dried to yield 11.82 g of the desired 9 - amino - 1,2,3,4 - tetrahydroacridin - 2 - ol. A portion of the product was converted to the maleate in MeOH and recrystallized from MeOH/$Et_2O$, mp 225° (d).

| ANALYSIS: | | | |
|---|---|---|---|
| Calculated for $C_{13}H_{14}N_2O \cdot C_4H_4O_4$:<br>Found: | 61.81%C<br>61.64%C | 5.49%H<br>5.61%H | 8.48%N<br>8.42%N |

EXAMPLE 3

2 - Hydroxy - N - (dimethylaminomethylene) - 1,2,3,4 - tetrahydro - 9 - acridinamine

9 - Amino - 1,2,3,4 - tetrahydroacridin - 2 - ol (10.97 g) was refluxed in dimethylformamide dimethyl acetal (329 ml) for two hours. The reaction mixture was cooled to room temperature, diluted with saturated $K_2CO_3$ (1 liter), extracted with ethyl acetate (2x 600 ml), stirred in activated carbon for a half hour, filtered through celite, dried ($MgSO_4$), and concentrated to obtain a solid. The solid was triturated in diethyl ether to yield 5.68 g of product. Recrystallization from methylene chloride/hexane and drying under high vacuum with refluxing 3 - hexanone for twenty - four hours yielded 4.37 g of a solid, mp 158° (dec.).

| ANALYSIS: | | | |
|---|---|---|---|
| Calculated for $C_{16}H_{19}N_3O$:<br>Found: | 71.35%C<br>70.86%C | 7.11%H<br>6.90%H | 15.60%N<br>15.40%N |

EXAMPLE 4

2 - Acetoxy - 1,2,3,4 - tetrahydro - 9 - acridinamine

2 - Benzyloxy - 1,2,3,4 - tetrahydro - 9 - acridinamine (10.97 g) was suspended in 500 ml of liquid $NH_3$ at -65°. Sodium was then added in small chucks until the blue color persisted (used 2.2 g). The reaction was quenched by the addition of solid $NH_4Cl$, and then the $NH_3$ was allowed to evaporate and the residue was washed well with ethyl acetate and filtered off. The ethyl acetate wash was concentrated and shown to contain 2 - acetoxy - 1,2,3,4 - tetrahydro - 9 - acridinamine. This was flushed over a silica gel column (5% $Et_3N$/EtOAc) and then the product - containing fractions were concentrated and the residue converted to the hydrochloride in ethereal HCl. Recrystallization from MeOH/$ET_2O$ gave 2.30 g, mp 178 - 181°.

| ANALYSIS: | | | |
|---|---|---|---|
| Calculated for $C_{15}H_{16}N_2O_2 \cdot HCl$: | 61.53%C | 5.85%H | 9.57%N |
| Found: | 60.88%C | 5.71%H | 9.38%N |

EXAMPLE 5

2-Propionyloxy-1,2,3,4-tetrahydro-9-acridinamine, fumarate hemihydrate

To a suspension of 9-amino-1,2,3,4-tetrahydroacridin-2-ol hydrochloride (5.0 g) in pyridine (6.15 ml) and dry N,N-dimethylformamide (240 ml) was added propionyl chloride (2.17 ml) dropwise. The reaction mixture was stirred at room temperature for two hours, an additional 1.0 ml of propionyl chloride was added and the reaction mixture was maintained at 45°C for sixty-four hours.

The reaction mixture was cooled to room temperature, diluted with saturated $NaHCO_3$ (400 ml) and deionized water (400 ml), and extracted with ethyl acetate (5x 300 ml). The organics were dried ($MgSO_4$) and concentrated to yield 4.03 g of crude product. Purification via trituration ($Et_2O$), flash chromatography (5% $Et_3N$/EtOAc), and recrystallization from ethyl acetate yielded 1.88 g of product.

This was combined with an additional run and the fumarate salt was made using isopropanol as the solvent. Recrystallization from ethyl acetate and drying under high vacuum with refluxing xylenes for thirty-six hours yielded 2.7 g of off-white microneedles, mp 220° (dec.).

| ANALYSIS: | | | |
|---|---|---|---|
| Calculated for $C_{16}H_{18}N_2O_2 \cdot C_4H_4O_4 \cdot 0.5H_2O$: | 60.75%C | 5.86%H | 7.09%N |
| Found: | 60.98%C | 5.47%H | 7.11%N |

EXAMPLE 6

2-Butyryloxy-1,2,3,4-tetrahydro-9-acridinamine, fumarate

To a suspension of 9-amino-1,2,3,4-tetrahydroacridin-2-ol hydrochloride (8.0 g) in pyridine (11.16 ml) and dry N,N-dimethylformamide (376 ml) was added butyryl chloride (4.67 ml). The reaction mixture was stirred in a pre-heated oil bath (80°C) for a half hour, an additional 4.67 ml of butyryl chloride was added and heating was continued at 80°C for fifteen minutes. The reaction mixture was cooled to room temperature, diluted with saturated $K_2CO_3$ (1 liter) and extracted with ethyl acetate (2x 700 ml), and the organic phase was washed with water (2x 500 ml), dried ($MgSO_4$), and concentrated to obtain an oil.

Purification via flash chromatography (1.5% $Et_3N$/EtOAc) and trituration ($Et_2O$/hexane (1:1)) yielded 2.67 g of product. The fumarate was made using isopropanol as the solvent. Recrystallization from ethanol/hexane yielded 2.74 g of white solid, mp 222°C (dec.).

| ANALYSIS: | | | |
|---|---|---|---|
| Calculated for $C_{17}H_{20}N_2O_2 \cdot C_4H_4O_4$: | 62.99%C | 6.04%H | 7.00%N |
| Found: | 62.66%C | 5.96%H | 6.98%N |

EXAMPLE 7

2-Valeryloxy-1,2,3,4-tetrahydro-9-acridinamine, fumarate

To a suspension of 9-amino-1,2,3,4-tetrahydroacridin-2-ol hydrochloride (8.0 g) in pyridine (11.16 ml) and dry N,N-dimethylformamide (376 ml) was added valeryl chloride (5.43 ml). The reaction mixture was stirred in a pre-heated oil bath (80°C) for a half hour, an additional 5.43 ml of valeryl chloride was added and heating was continued at 80°C for twenty minutes. The reaction mixture was cooled to room temperature, diluted with saturated $K_2CO_3$ (1 liter) and extracted with ethyl acetate (750 ml), and the organic

phase was washed with water (2x 350 ml), dried ($MgSO_4$), and concentrated to obtain an oil.

Purification via flash chromatography (1.5% $Et_3N$/EtOAc) and trituration ($Et_2O$/hexane (1:3)) yielded 3.85 g (41%) of product. The fumarate was made using isopropanol as the solvent. Recrystallization from methanol/diethyl ether and drying under high vacuum with refluxing xylenes for forty-eight hours yielded 4.54 g of a solid, mp 210°C (dec.).

| ANALYSIS: | | | |
|---|---|---|---|
| Calculated for $C_{18}H_{22}N_2O_2 \cdot C_4H_4O_4$: | 63.75%C | 6.32%H | 6.76%N |
| Found: | 63.53%C | 6.31%H | 6.71%N |

EXAMPLE 8

2-Hexanoyloxy-1,2,3,4-tetrahydro-9-acridinamine, fumarate

To a suspension of 9-amino-1,2,3,4-tetrahydroacridin-2-ol hydrochloride (3.0 g) in pyridine (3.63 ml) and dry N,N-dimethylformamide (141 ml) was added hexanoyl chloride (2.19 ml) dropwise. The reaction mixture was stirred in a pre-heated oil bath (80°C) for one hour, an additional 2.19 ml of hexanoyl chloride was added, heating was continued for one hour. and the reaction mixture was cooled to room temperature.

The reaction mixture was diluted with saturated $K_2CO_3$ (600 ml) and extracted with ethyl acetate (400 ml), and the organic phase was washed with water (2x 250 ml), dried ($MgSO_4$), and concentrated to obtain an oil.

This was combined with an additional run and purified via flash chromatography (1.5% $ET_3N$/EtOAc). Trituration ($Et_2O$) yielded 2.35 g of product. The fumarate was made using isopropanol (50 ml) as the solvent. Recrystallization from ethanol/diethyl ether and drying under high vacuum with refluxing xylenes for fifty-two hours yielded 2.41 g of microcrystals, mp 205°C (dec.).

| ANALYSIS: | | | |
|---|---|---|---|
| Calculated for $C_{19}H_{24}N_2O_2 \cdot C_4H_4O_4$: | 64.47%C | 6.59%H | 6.54%N |
| Found: | 64.34%C | 6.45%H | 6.55%N |

EXAMPLE 9

2-Heptanoyloxy-1,2,3,4-tetrahydro-9-acridinamine, fumarate

To a suspension of 9-amino-1,2,3,4-tetrahydroacridin-2-ol hydrochloride (5.0 g) in pyridine (6.93 ml) and dry N,N-dimethylformamide (235 ml) was added heptanoyl chloride (4.32 ml) dropwise. The reaction mixture was stirred in a pre-heated oil bath (80°C) for a half hour, an additional 4.32 ml of heptanoyl chloride was added, heating was continued for five hours, and thereafter the reaction mixture was stirred at room temperature overnight.

The reaction mixture was diluted with saturated $K_2CO_3$ (750 ml) and extracted with ethyl acetate (660 ml), and the organic phase was washed with water (700 ml), dried ($MgSO_4$) and concentrated to obtain an oil. Purification via flash chromatography (2.5% $ET_3N$/EtOAc) and trituration ($Et_2O$/Hexane (1:1)) yielded 2.65 g of product. The fumarate was made using isopropanol (30 ml) as the solvent. Recrystallization from ethanol/hexane and drying under high vacuum with refluxing n-butyl acetate for sixteen hours yielded 2.49 g of microcrystals, mp 195°C (dec.).

| ANALYSIS: | | | |
|---|---|---|---|
| Calculated for $C_{20}H_{26}N_2O_2 \cdot C_4H_4O_4$: | 65.14%C | 6.83%H | 6.33%N |
| Found: | 64.86%C | 6.63%H | 6.24%N |

EXAMPLE 10

2-Octanoyloxy-1,2,3,4-tetrahydro-9-acridinamine, fumarate

To a suspension of 9-amino-1,2,3,4-tetrahydroacridin-2-ol hydrochloride (8.0 g) in pyridine (11.16 ml) and dry N,N-dimethylformamide (376 ml) was added octanoyl chloride (7.68 ml) dropwise. The reaction mixture was stirred in a pre-heated oil bath (80°C) for a half hour, an additional 7.68 ml of octanoyl chloride was added and heating was continued at 80°C for a half hour. The reaction mixture was cooled to room temperature, diluted with saturated $K_2CO_3$ (1 liter) and extracted with ethyl acetate (800 ml), and the organic phase was washed with water (2x 250 ml), dried ($MgSO_4$) and concentrated to obtain an oil which solidified overnight.

Purification via trituration (pentane/diethyl ether) yielded 2.85 g of product. The fumarate was made using isopropanol as the solvent. Recrystallization from methanol/diethyl ether and drying under high vacuum with refluxing xylenes for nineteen hours yielded 2.87 g of a white solid, mp 199°C (dec.).

| ANALYSIS: | | | |
|---|---|---|---|
| Calculated for $C_{21}H_{28}N_2O_2 \cdot C_4H_4O_4$: | 65.77%C | 7.07%H | 6.14%N |
| Found: | 65.68%C | 7.16%H | 6.15%N |

EXAMPLE 11

2-Nonanoyloxy-1,2,3,4-tetrahydro-9-acridinamine, fumarate

To suspension of 9-amino-1,2,3,4-tetrahydroacridin-2-ol hydrochloride (4.0 g) in pyridine (5.56 ml) and dry N,N-dimethylformamide (188 ml) was added nonanoyl chloride (4.0 ml) dropwise. The reaction mixture was stirred in a pre-heated oil bath (80°C) for one hour, an additional 4.0 ml of nonanoyl chloride was added, heating was continued for a half hour, and the reaction mixture was cooled to room temperature.

The reaction mixture was combined with 1.0 g of probe reaction, diluted with saturated $K_2CO_3$ (1 liter) and extracted with ethyl acetate (750 ml), and the organic phase was washed with water (2x 500 ml), dried ($MgSO_4$) and concentrated to obtain an oil. Purification via flash chromatography (1.5% $Et_3N$/EtOAc) and trituration (pentane/$Et_2O$) yielded 2.02 g of product. This was combined with an additional 3.0 g run and the fumarate was made using isopropanol as the solvent. Recrystallization from ethanol/hexane and drying under high vacuum with refluxing xylenes overnight yielded 3.0 g of a white solid, mp 203°C (dec.).

| ANALYSIS: | | | |
|---|---|---|---|
| Calculated for $C_{22}H_{30}N_2O_2 \cdot C_4H_4O_4$: | 66.36%C | 7.28%H | 5.95%N |
| Found: | 66.23%C | 7.19%H | 5.91%N |

EXAMPLE 12

2-Decanoyloxy-1,2,3,4-tetrahydro-9-acridinamine, fumarate

To a suspension of 9-amino-1,2,3,4-tetrahydroacridin-2-ol hydrochloride (5.31 g) in pyridine (7.28 ml) and dry N,N-dimethylformamide (247 ml) was added decanoyl chloride (6.02 ml) dropwise. The reaction mixture was stirred in a pre-heated oil bath (45°C) for a half hour, an additional 6.02 ml of decanoyl chloride was added and heating was continued at 45°C overnight. The reaction mixture was heated to 80°C for one hour, cooled to room temperature, diluted with saturated $K_2CO_3$ (700 ml), extracted with ethyl acetate (600 ml), washed with deionized water (500 ml), dried ($MgSO_4$) and concentrated to obtain an oil. Purification via flash chromatography (2.5% $Et_3N$/hexane) and trituration ($Et_2O$/hexane (1:3)) yielded 3.67 g of product. The fumarate was made using isopropanol as the solvent. Recrystallization from ethanol/hexane and drying under high vacuum with refluxing xylenes overnight yielded 3.8 g of microcrystals, mp 212°C (dec.).

| ANALYSIS: | | | |
|---|---|---|---|
| Calculated for $C_{23}H_{32}N_2O_2 \cdot C_4H_4O_4$:<br>Found: | 66.92%C<br>67.10%C | 7.49%H<br>7.47%H | 5.78%N<br>5.73%N |

EXAMPLE 13

2-Lauroyloxy-1,2,3,4-tetrahydro-9-acridinamine

To a suspension of 9-amino-1,2,3,4-tetrahydroacridin-2-ol hydrochloride (6.0 g) in pyridine (8.32 ml) and dry N,N-dimethylformamide (282 ml) was added lauroyl chloride (7.74 ml) dropwise. The reaction mixture was stirred in a pre-heated oil bath (80°C) for a half hour, an additional 7.74 ml of lauroyl chloride was added, heating was continued for one hour, and thereafter the reaction mixture was stirred at room temperature overnight.

The reaction mixture was diluted with saturated $K_2CO_3$ (1200 ml), extracted with ethyl acetate (800 ml), washed with water (500 ml), dried ($MgSO_4$) and concentrated to obtain a gummy solid. The solid was purified via flash chromatography (2.5% $Et_3N$/EtOAc) and trituration ($Et_2O$/hexane (1:1)) to yield 5.25 g of product. Recrystallization from $Et_2O$/hexane/EtOH and drying under high vacuum with refluxing ethanol for eighteen hours yielded 4.0 g of solid, mp 115°C (dec.).

| ANALYSIS: | | | |
|---|---|---|---|
| Calculated for $C_{25}H_{36}N_2O_2$:<br>Found: | 75.72%C<br>75.60%C | 9.15%H<br>9.19%H | 7.06%N<br>7.10%N |

EXAMPLE 14

4-Acetoxy-9-chloro-1,2,3,4-tetrahydroacridine

9-Chloro-1,2,3,4-tetrahydroacridine N-oxide (40.12 g) was refluxed for thirty minutes in 200 ml of acetic anhydride. At the end of this time the acetic anhydride was evaporated and the reaction mixture was purified by preparative HPLC (20% EtOAc/hexane, two columns, two runs). Concentration of the product-containing fractions and trituration with pentane gave 33.1 g of product, mp 83-85°C. Analytically pure material was obtained by recrystallization from hexane, mp 85-87°C.

| ANALYSIS: | | | |
|---|---|---|---|
| Calculated for $C_{13}H_{14}ClNO_2$:<br>Found: | 65.33%C<br>65.51%C | 5.12%H<br>5.15%H | 5.08%N<br>5.07%N |

EXAMPLE 15

4-Benzyloxy-9-chloro-1,2,3,4-tetrahydroacridine

9-Chloro-4-hydroxy-1,2,3,4-tetrahydroacridine (20.78 g) was dissolved in 500 ml of dimethylformamide to which benzyl bromide (30.8 g) was then added. Sodium hydride (4.80 g of 50% suspension) was added and the reaction mixture was stirred for two hours at room temperature. At the end of this time the reaction mixture was distributed between $Et_2O$ and $H_2O$ and then the organic phase was separated, washed well with $H_2O$, dried and concentrated. The residue was triturated with pentane to give 19.60 g of product, mp 73-75°C. The pentane wash was shown by TLC to still contain a considerable amount of product, and so it was further purified by flash chromatography (10% EtOAc/hexane) to give an additional 7.42 g of product, mp 74-75°C. Material of analytical purity was obtained by recrystallization from pentane, mp 75-76°C.

| ANALYSIS: | | | |
|---|---|---|---|
| Calculated for $C_{20}H_{18}ClNO_2$: | 74.18%C | 5.60%H | 4.33%N |
| Found: | 74.12%C | 5.57%H | 4.29%N |

EXAMPLE 16

4-Benzyloxy-1,2,3,4-tetrahydro-9-acridinamine

4-Benzyloxy-9-chloro-1,2,3,4-tetrahydroacridine (24.0 g) was dissolved in 150 ml of phenol at 130°C and $NH_3$ was passed into the solution for two hours. At the end of this time the reaction mixture was distributed between $Et_2O$ and 10% NaOH and then the organic phase was washed again with 10% NaOH and then with $H_2O$. The organic phase was dried, concentrated, and then purified by flash chromatography (EtOAc). Concentration of the product-containing fractions and trituration of the residue with $Et_2O$ gave 10.12 g of product.

| ANALYSIS: | | | |
|---|---|---|---|
| Calculated for $C_{20}H_{20}N_2O$: | 78.92%C | 6.62%H | 9.20%N |
| Found: | 78.67%C | 6.52%H | 9.09%N |

EXAMPLE 17

9-Amino-1,2,3,4-tetrahydroacridinamin-4-ol, hemifumarate

4-Benzyloxy-1,2,3,4-tetrahydro-9-acridinamine (6.0 g) was refluxed for one hour in a mixture of 50 ml glacial acetic acid and 50 ml concentrated HCl. At the end of this time the reaction mixture was concentrated under reduced pressure and the residue purified by flash chromatography (5% $Et_3N$/EtOAc) to give 2.83 g of free base. The fumarate was formed in isopropanol and recrystallized from water to give 2.63 g of the hemi-fumarate, mp 225°C (dec.).

| ANALYSIS: | | | |
|---|---|---|---|
| Calculated for $C_{13}H_{14}N_2O \cdot 0.5C_4H_4O_4$: | 66.16%C | 5.92%H | 10.29%N |
| Found | 65.80%C | 5.88%H | 10.16%N |

EXAMPLE 18

6-Chloro-2-benzyloxy-1,2,3,4-tetrahydro-9-acridinamine

To a mechanically stirred solution of 4-benzyloxycyclohexanone[1] (10.77 g) and 4-chloroanthranilonitrile[2] (7.93 g) in nitrobenzene (72.6 ml) was added freshly fused zinc chloride (7.9 g). The reaction mixture was heated at 60°C for 90 minutes and thereafter at 120°C for three hours. The reaction mixture was cooled to room temperature, anhydrous diethyl ether (170 ml) was added dropwise, and the precipitate was filtered, washed well with diethyl ether and dissolved in methanol (200 ml). Saturated ammonium hydroxide (200 ml) was added dropwise while the mixture was cooled in an ice bath. The resulting solid was filtered, washed successively with deionized water and diethyl ether and air dried to yield 7.2 g of a white solid. A 4.86 g portion was recrystallized from ethanol/hexane and dried under high vaccum with the aid of refluxing xylenes to yield 3.5 g of product; mp 220° (dec.).

[1]J. Chem. Soc., Chem Commum. 1984, 721.

[2]J. Amer. Chem. Soc. 1947, Volume 69, 940.

| ANALYSIS: | | | |
|---|---|---|---|
| Calculated for $C_{20}H_{19}N_2OCl$:<br>Found | 70.90%C<br>70.71%C | 5.65%H<br>5.60%H | 8.27%N<br>8.21%N |

EXAMPLE 19

4 – Acetoxy – 1,2,3,4 – tetrahydro – 9 – acridinamine fumarate, hemihydrate

9 – Amino – 1,2,3,4 – tetrahydroacridin – 4 – ol (2.40 g) was refluxed for two hours in 20 ml of acetic anhydride. The reaction mixture was then concentrated and distributed between 2 – butanone and $NaHCO_3$ solution. Concentration of the organic phase gave a residue that was triturated with $Et_2O$, giving a crude product as a free base. The fumarate was formed in isopropanol, amounting to 1.82 g, which analyzed for the fumarate hemihydrate, mp 258° (dec.).

| ANALYSIS: | | | |
|---|---|---|---|
| Calculated for $C_{15}H_{16}N_2O_2 \cdot C_4H_4O_4 \cdot 0.5\ H_2O$:<br>Found | 59.83%C<br>59.59%C | 5.55%H<br>5.71%H | 7.35%N<br>6.99%N |

**Claims**

**Claims for the following Contracting States : AT, BE, CH, DE, FR, GB, IT, LI, LU, NL, SE**

**1.** A compound of the formula I

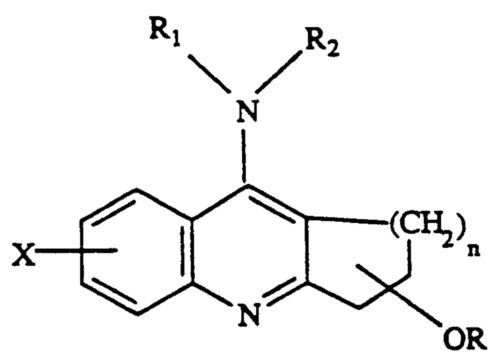

(I)

where
n is 2;
X is hydrogen or halogen,
R is hydrogen, benzyl or alkanoyl of 2 – 12 carbon atoms, with the proviso that the group – OR may not be on the 1 – position of the three ring system, or if R is hydrogen, the group – OR may not be on the 4 – position of the three ring system;
$R_1$ and $R_2$ are each independently hydrogen, $C_1$ – $C_6$ – alkyl or phenyl – $C_1$ – $C_6$ – alkyl wherein phenyl is optionally substituted with 1,2 or 3 substituents each of which being independently $C_1$ – $C_6$ – alkyl, $C_1$ – $C_6$ – alkoxy, halogen, hydroxy or trifluoromethyl, but both may not be phenyl – $C_1$ – $C_6$ – alkyl simultaneously, or alternatively $R_1$ and $R_2$ taken together may constitute the group

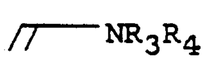

where $R_3$ and $R_4$ are each independently $C_1$ – $C_6$ – alkyl; or
a stereoisomer thereof or a pharmaceutically acceptable acid addition salt thereof.

**2.** A compound as defined in claim 1 where the group OR is in 2 – position.

**3.** A compound as defined in claim 2, wherein $R_1$, $R_2$ and X are hydrogen.

**4.** A compound as defined in claim 3, wherein R is hydrogen or $C_2 - C_6$ – alkanoyl.

**5.** The compound as defined in claim 1, which is 9 – amino – 1,2,3,4 – tetrahydroacridin – 2 – ol, or a pharmaceutically acceptable acid addition salt thereof.

**6.** The compound as defined in claim 1, which is 2 – acetoxy – 1,2,3,4 – tetrahydro – 9 – acridinamin, or a pharmaceutically acceptable acid addition salt thereof.

**7.** The compound as defined in claim 1, which is 2 – butyryloxy – 1,2,3,4 – tetrahydro – 9 – acridinamin, or a pharmaceutically acceptable acid addition salt thereof.

**8.** The compound as defined in claim 1, which is 2 – hexanoyloxy – 1,2,3,4 – tetrahydro – 9 – acridinamin, or a pharmaceutically acceptable acid addition salt thereof.

**9.** A pharmaceutical composition which comprises as the active ingredient a compound as defined in claim 1 and a suitable carrier therefor.

**10.** Use of a compound as defined in claim 1 for the preparation of a medicament having memory dysfunction alleviating activity.

**11.** A process for the preparation of a compound as defined in claim 1, which comprises
a) reacting a compound of the formula

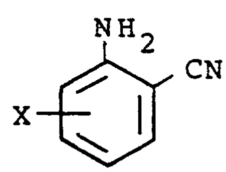

$a_1$) with a compound of the formula

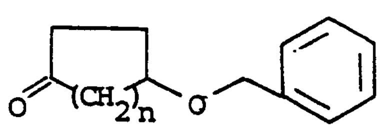

wherein n is 2, in the presence of a Lewis acid, to obtain a compound of the formula I'

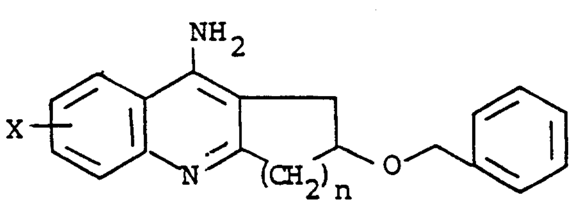

I'

wherein X and n are as defined, or

$a_2$) with a compound of the formula

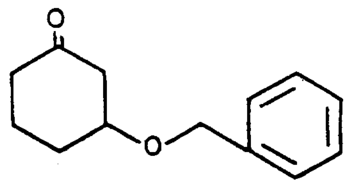

in the presence of a Lewis acid, to obtain a compound of the formula I"

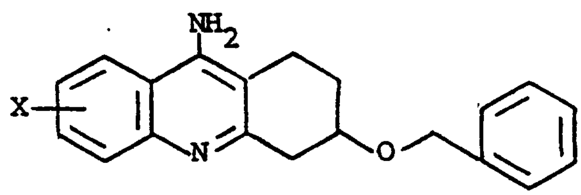

I"

wherein X is as defined, or

b) reacting a compound of the formula XVIII

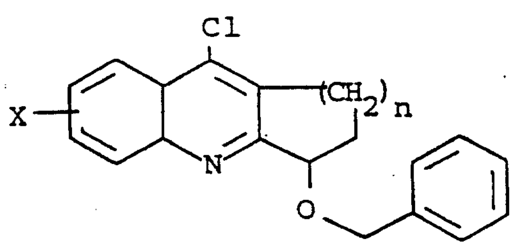

XVIII

wherein X and n are as defined,
with ammonia in a suitable medium to obtain a compound of the formula I'''

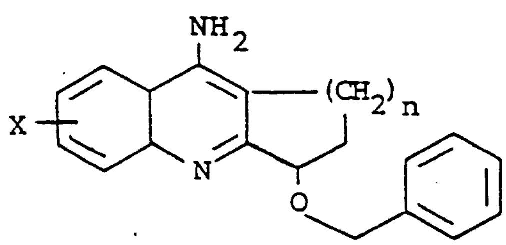

I'''

c) optionally reacting a compound of the formulae I', I" or I''' above, with a compound of the formula $R_1W$, where W is Cl, Br, I or $-OSO_3CH_3$ and $R_1$ is as defined above, to afford a compound of the formula I, wherein R is benzyl, $R_1$ is as defined above, X is as defined above and n is 2,

d) optionally reacting a compound of the formula I wherein R is benzyl, $R_1$ is as defined above, $R_2$ is hydrogen, X is as defined above and n is 2, with a compound of the formula $R_2W$, where W is as defined in step c) and $R_2$ is as defined above, to obtain a compound of the formula I, wherein R is benzyl and $R_1$, $R_2$, X and n are as defined,

e) optionally subjecting a compound of the formula I, wherin the OR group is in the 2- or 3-position, and R is benzyl, $R_1$ and $R_2$ are hydrogen, $C_1-C_6$-alkyl or phenyl-$C_1-C_6$-alkyl wherein the phenyl is optionally substituted with 1,2 or 3 substituents each of which being independently $C_1-C_6$-alkyl, $C_1-C_6$-alkoxy, halogen, hydroxy or trifluoromethyl, X and n are as defined above to hydrogenolysis to obtain a compound of the formula I, where R is hydrogen, or

f) optionally reacting a compound of the formula I, where R is hydrogen, $R_1$, $R_2$, X and n are as defined above, with an acyl-chloride of the formula $R_5COCl$ or an ester of the formula

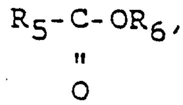

where $R_5$ is $C_1-C_{11}$-alkyl and $R_6$ is $C_1-C_2$-alkyl, to obtain a compound of the formula I, wherein R is $C_2-C_{12}$-alkanoyl, and $R_1$, $R_2$, X and n are as defined above, and

g) optionally reacting a compound of the formula I,wherein R is hydrogen, $R_1$ and $R_2$ are hydrogen, $C_1-C_6$-alkyl or phenyl-$C_1-C_6$-alkyl, wherein the phenyl is optionally substituted with 1,2 or 3 substituents each of which being independently $C_1-C_6$-alkyl, $C_1-C_6$-alkoxy, halogen, hydroxy or trifluoromethyl, and X and n are as defined, with a formamide acetal of the formula VIII

$R_3R_4NCH(OR_7)_2$ VIII

wherein $R_3$, $R_4$, and $R_7$ are $C_1-C_6$-alkyl, to obtain a compound of the formula I, wherein $R_1$ and $R_2$ together constitute the group $=CH-NR_3R_4$, where $R_3$ and $R_4$ are as defined.

**Claims for the following Contracting States : ES, GR**

**1.** A process for the preparation of a compound of the formula I

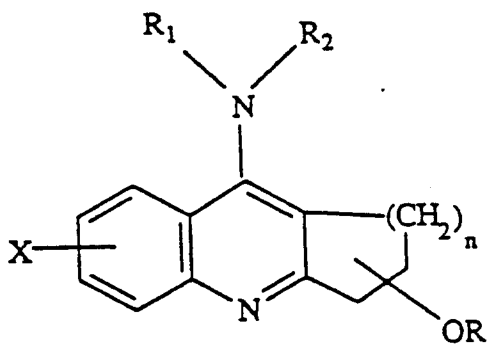

(I)

where
n is 2;
X is hydrogen or halogen,
R is hydrogen, benzyl or alkanoyl of 2-12 carbon atoms, with the proviso that the group -OR may not be on the 1-position of the three ring system, or if R is hydrogen, the group -OR may not be on the 4-position of the three ring system;
$R_1$ and $R_2$ are each independently hydrogen, $C_1-C_6$-alkyl or phenyl-$C_1-C_6$-alkyl wherein phenyl is optionally substituted with 1,2 or 3 substituents each of which being independently $C_1-C_6$-alkyl, $C_1-C_6$-alkoxy, halogen, hydroxy or trifluoromethyl, but both may not be phenyl-$C_1-C_6$-alkyl simultaneously, or alternatively $R_1$ and $R_2$ taken together may constitute the group

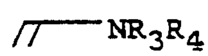

where $R_3$ and $R_4$ are each independently loweralkyl; or
a stereoisomer thereof or a pharmaceutically acceptable acid addition salt thereof,
which comprises

a) reacting a compound of the formula

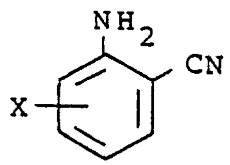

$a_1$) with a compound of the formula

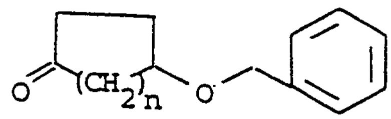

wherein n is 2, in the presence of a Lewis acid, to obtain a compound of the formula I'

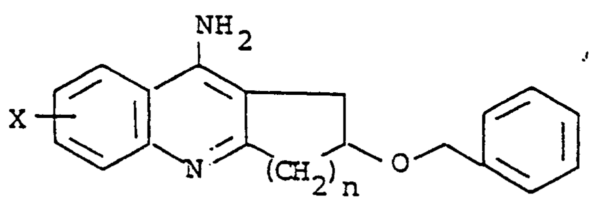

I'

wherein X and n are as defined, or
$a_2$) with a compound of the formula

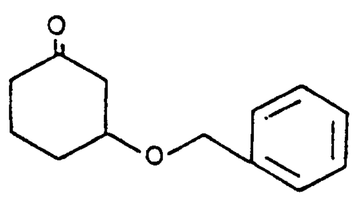

in the presence of a Lewis acid, to obtain a compound of the formula I"

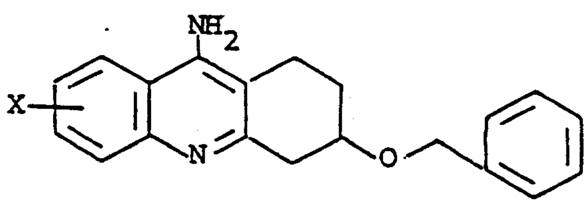

I"

wherein X is as defined, or
b) reacting a compound of the formula XVIII

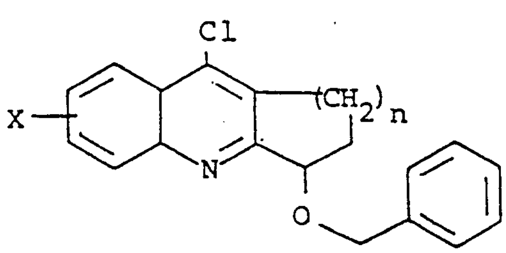

XVIII

wherein X and n are as defined,
with ammonia in a suitable medium to obtain a compound of the formula I'''

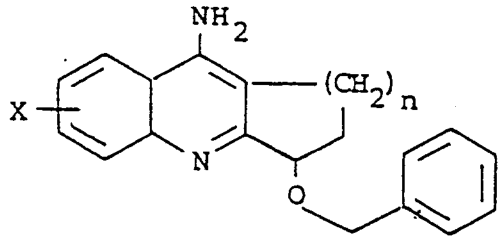

I'''

c) optionally reacting a compound of the formulae I', I" or I''' above, with a compound of the formula $R_1W$, where W is Cl, Br, I or $-OSO_3CH_3$ and $R_1$ is as defined above, to afford a compound of the formula I, wherein R is benzyl, $R_1$ is as defined above, X is as defined above and n is 2,
d) optionally reacting a compound of the formula I wherein R is benzyl, $R_1$ is as defined above, $R_2$ is hydrogen, X is as defined above and n is 2, with a compound of the formula $R_2W$, where W is as defined in step c) and $R_2$ is as defined above, to obtain a compound of the formula I, wherein R is benzyl and $R_1$, $R_2$, X and n are as defined
e) optionally subjecting a compound of the formula I, wherin the OR group is in the 2- or 3-position, and R is benzyl, $R_1$ and $R_2$ are hydrogen, $C_1-C_6$-alkyl or phenyl-$C_1-C_6$-alkyl wherin phenyl is optionally substituted with 1,2 or 3 substituents each of which being independently $C_1-C_6$-alkyl, $C_1-C_6$-alkoxy, halogen, hydroxy or trifluoromethyl, X and n are as defined above to hydrogenolysis to obtain a compound of the formula I, where R is hydrogen, or
f) optionally reacting a compound of the formula I, where R is hydrogen, $R_1$, $R_2$, X and n are as defined above, with an acyl-chloride of the formula $R_5COCl$ or an ester of the formula

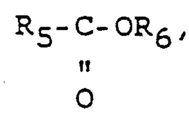

where $R_5$ is $C_1-C_{11}$-alkyl and $R_6$ is $C_1-C_2$-alkyl, to obtain a compound of the formula I, wherein R is $C_2-C_{12}$-alkanoyl, and $R_1$, $R_2$, X and n are as defined above, and
g) optionally reacting a compound of the formula I,wherein R is hydrogen, $R_1$ and $R_2$ are hydrogen, $C_1-C_6$-alkyl or phenyl-$C_1-C_6$-alkyl, wherein phenyl is optionally substituted with 1,2 or 3 substituents each of which being independently $C_1-C_6$-alkyl, $C_1-C_6$-alkoxy, halogen, hydroxy or trifluoromethyl, and X and n are as defined, with a formamide acetal of the formula VIII

$R_3R_4NCH(OR_7)_2$ VIII

wherein $R_3$, $R_4$ and $R_7$ are $C_1-C_6$-alkyl, to obtain a compound of the formula I, wherein $R_1$ and $R_2$ together constitute the group $=CH-NR_3R_4$, where $R_3$ and $R_4$ are as defined.

**2.** A process as defined in claim 1, where n is 2, and the group OR is in 2- position.

**3.** A process as defined in claim 2, wherein $R_1$, $R_2$ and X are hydrogen.

**4.** A process as defined in claim 3, wherein R is hydrogen or $C_2-C_6$-alkanoyl.

**5.** The process as defined in claim 1, wherein 9-amino-1,2,3,4-tetrahydroacridin-2-ol, or a pharmaceutically acceptable acid addition salt thereof is prepared.

**6.** The process as defined in claim 1, wherein 2-acetoxy-1,2,3,4-tetrahydro-9-acridinamin, or a pharmaceutically acceptable acid addition salt thereof is prepared.

**7.** The process as defined in claim 1, wherein 2-butyryloxy-1,2,3,4-tetrahydro-9-acridinamin, or a pharmaceutically acceptable acid addition salt thereof is prepared.

**8.** The process as defined in claim 1, wherein 2-hexanoyloxy-1,2,3,4-tetrahydro-9-acridinamin, or a pharmaceutically acceptable acid addition salt thereof is prepared.

**9.** Use of a compound as defined in claim 1 for the preparation of a medicament having memory dysfunction alleviating activity.

**Patentansprüche**
**Patentansprüche für folgende Vertragsstaaten : AT, BE, CH, DE, FR, GB, IT, LI, LU, NL, SE**

**1.** Eine Verbindung mit der Formel I

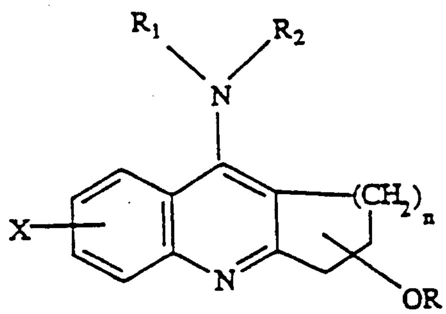

(I),

worin
n den Wert 2 hat;
X Wasserstoff oder Halogen bedeutet;
R für Wasserstoff, Benzyl oder Alkanoyl mit 2 bis 12 Kohlenstoffatomen steht, mit der Maßgabe, daß die Gruppe -OR nicht an der 1-Stellung des Dreiringsystems vorliegen kann, oder, falls R für Wasserstoff steht, die Gruppe -OR nicht an der 4-stellung des Dreiringsystems vorliegen kann;
$R_1$ und $R_2$ jeweils unabhängig voneinander Wasserstoff, $C_1-C_6$-Alkyl oder Phenyl-$C_1-C_6$-alkyl bedeuten, worin Phenyl gegebenenfalls durch 1, 2 oder 3 Substituenten substituiert ist, die jeweils unabhängig voneinander $C_1-C_6$-Alkyl, $C_1-C_6$-Alkoxy, Halogen, Hydroxy oder Trifluormethyl sind, die Reste $R_1$ und $R_2$ aber nicht beide gleichzeitig Phenyl-$C_1-C_6$-alkyl sein können, oder alternativ $R_1$ und $R_2$ zusammen die Gruppe

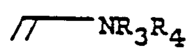

ausbilden können, worin $R_3$ und $R_4$ jeweils unabhängig voneinander $C_1-C_6$-Alkyl bedeuten;
oder ein Stereoisomer hievon oder ein pharmazeutisch annehmbares Säureadditionssalz hievon.

**2.** Verbindung nach Anspruch 1, worin die Gruppe -OR in der 2-Stellung vorliegt.

**3.** Verbindung nach Anspruch 2, worin $R_1$, $R_2$ und X Wasserstoff bedeuten.

**4.** Verbindung nach Anspruch 3, worin R Wasserstoff oder $C_2-C_6$-Alkanoyl bedeutet.

**5.** Verbindung nach Anspruch 1, nämlich 9-Amino-1,2,3,4-tetrahydroacridin-2-ol oder ein pharmazeutisch annehmbares Säureadditionssalz hievon.

**6.** Verbindung nach Anspruch 1, nämlich 2-Acetoxy-1,2,3,4-tetrahydro-9-acridinamin oder ein pharmazeutisch annehmbares Säureadditionssalz hievon.

**7.** Verbindung nach Anspruch 1, nämlich 2-Butyryloxy-1,2,3,4-tetrahydro-9-acridinamin oder ein pharmazeutisch annehmbares Säureadditionssalz hievon.

**8.** Verbindung nach Anspruch 1, nämlich 2-Hexanoyloxy-1,2,3,4-tetrahydro-9-acridinamin oder ein pharmazeutisch annehmbares Säureadditionssalz hievon.

**9.** Pharmazeutische Zusammensetzung, die als wirksamen Bestandteil eine Verbindung gemäß Definition im Anspruch 1 und einen hiefür geeigneten Träger umfaßt.

**10.** Verwendung einer Verbindung, wie in Anspruch 1 definiert, zur Herstellung eines Medikaments mit einer die Gedächtnisdysfunktion mildernden Aktivität.

**11.** Verfahren zur Herstellung einer Verbindung nach Anspruch 1, welches umfaßt:
a) Umsetzen einer Verbindung der Formel

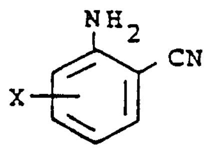

$a_1$) mit einer Verbindung der Formel

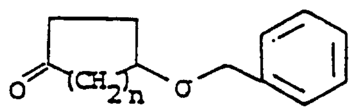

worin n den Wert 2 hat, in Anwesenheit einer Lewis-Säure zur Ausbildung einer Verbindung der Formel I'

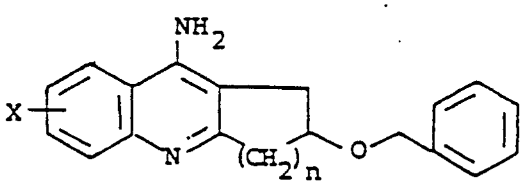

I' ,

worin X und n wie vorstehend definiert sind, oder
$a_2$) mit einer Verbindung der Formel

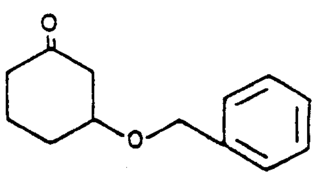

in Anwesenheit einer Lewis-Säure zur Ausbildung einer Verbindung der Formel I"

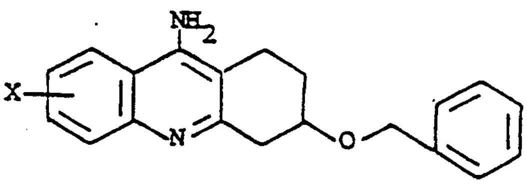

I",

worin X die angegebene Definition aufweist, oder

b) Umsetzen einer Verbindung der Formel XVIII

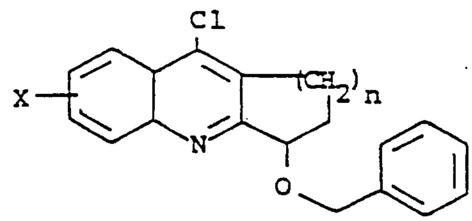

XVIII ,

worin X und n die angegebene Definition aufweisen, mit Ammoniak in einem geeigneten Medium zur Ausbildung einer Verbindung der Formel I'''

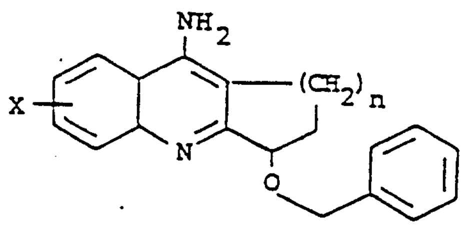

I''' ,

c) gegebenenfalls Umsetzen einer Verbindung der obigen Formeln I', I'' oder I''' mit einer Verbindung der Formel $R_1W$, worin W für Cl, Br, I oder $-OSO_3CH_3$ steht und $R_1$ die obige Definition aufweist, zur Ausbildung einer Verbindung der Formel I, worin R Benzyl bedeutet, $R_1$ wie oben definiert ist, X wie oben definiert ist und n den Wert 2 hat,
d) gegebenenfalls Umsetzen einer Verbindung der Formel I, worin R für Benzyl steht, $R_1$ die obige Definition aufweist, $R_2$ Wasserstoff darstellt, X wie oben definiert ist und n den Wert 2 hat, mit einer Verbindung der Formel $R_2W$, worin W wie in Stufe c) definiert ist und $R_2$ wie oben definiert ist, zur Ausbildung einer Verbindung der Formel I, worin R Benzyl bedeutet und $R_1$, $R_2$, X und n wie definiert sind,
e) gegebenenfalls Vornahme einer Hydrogenolyse an einer Verbindung der Formel I, worin die Gruppe $-OR$ in 2- oder 3-Stellung vorliegt und R Benzyl bedeutet, $R_1$ und $R_2$ für Wasserstoff, $C_1-C_6$-Alkyl oder Phenyl-$C_1-C_6$-alkyl stehen, worin das Phenyl gegebenenfalls durch 1, 2 oder 3 Substituenten substituiert ist, die jeweils unabhängig voneinander $C_1-C_6$-Alkyl, $C_1-C_6$-Alkoxy, Halogen, Hydroxy oder Trifluormethyl bedeuten, und X und n wie vorstehend definiert sind, unter Ausbildung einer Verbindung der Formel I, worin R für Wasserstoff steht, oder
f) gewünschtenfalls Umsetzen einer Verbindung der Formel I, worin R Wasserstoff bedeutet, $R_1$, $R_2$, X und n wie oben definiert sind, mit einem Acylchlorid der Formel $R_5COCl$ oder mit einem Ester der Formel

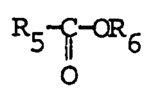

worin $R_5$ für $C_1-C_{11}$-Alkyl steht und $R_6$ für $C_1-C_2$-Alkyl steht, unter Ausbildung einer Verbindung der Formel I, worin R für $C_2-C_{12}$-Alkanoyl steht und $R_1$, $R_2$, X und n wie vorstehend definiert sind, und
g) gewünschtenfalls Umsetzen einer Verbindung der Formel I, worin R für Wasserstoff steht, $R_1$ und $R_2$ Wasserstoff, $C_1-C_6$-Alkyl oder Phenyl-$C_1-C_6$-alkyl bedeuten, worin das Phenyl gewünschtenfalls durch 1, 2 oder 3 Substituenten substituiert ist, die jeweils unabhängig voneinander $C_1-C_6$-Alkyl, $C_1-C_6$-Alkoxy, Halogen, Hydroxy oder Trifluormethyl sind, und X und n wie vorstehend definiert sind, mit einem Formamidacetal der Formel VIII

$R_3R_4NCH(OR_7)_2$ VIII ,

worin $R_3$, $R_4$ und $R_7$ für $C_1-C_6$-Alkyl stehen, unter Ausbildung einer Verbindung der Formel I,

worin $R_1$ und $R_2$ zusammen die Gruppe $=CH-NR_3R_4$ ausbilden, worin $R_3$ und $R_4$ wie vorstehend definiert sind.

**Patentansprüche für folgende Vertragsstaaten : ES, GR**

**1.** Verfahren zur Herstellung einer Verbindung mit der Formel I

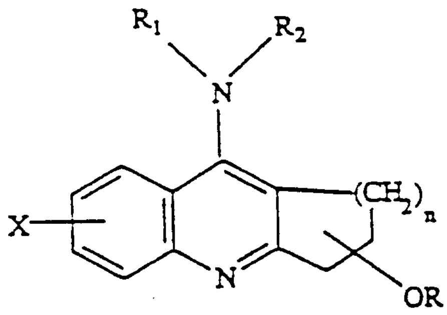

(I) ,

worin
n den Wert 2 hat;
X Wasserstoff oder Halogen bedeutet;
R für Wasserstoff, Benzyl oder Alkanoyl mit 2 bis 12 Kohlenstoffatomen steht, mit der Maßgabe, daß die Gruppe $-OR$ nicht an der 1-Stellung des Dreiringsystems vorliegen kann, oder, falls R für Wasserstoff steht, die Gruppe $-OR$ nicht an der 4-Stellung des Dreiringsystems vorliegen kann;
$R_1$ und $R_2$ jeweils unabhängig voneinander Wasserstoff, $C_1-C_6$-Alkyl oder Phenyl-$C_1-C_6$-alkyl bedeuten, worin Phenyl gegebenenfalls durch 1, 2 oder 3 Substituenten substituiert ist, die jeweils unabhängig voneinander $C_1-C_6$-Alkyl, $C_1-C_6$-Alkoxy, Halogen, Hydroxy oder Trifluormethyl sind, die Reste $R_1$ und $R_2$ aber nicht beide gleichzeitig Phenyl-$C_1-C_6$-alkyl sein können, oder alternativ $R_1$ und $R_2$ zusammen die Gruppe

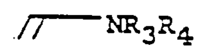

ausbilden können, worin $R_3$ und $R_4$ jeweils unabhängig voneinander $C_1-C_6$-Alkyl bedeuten;
oder eines Stereoisomers hievon oder eines pharmazeutisch annehmbaren Säureadditionssalzes hievon,
welches umfaßt:

a) Umsetzen einer Verbindung der Formel

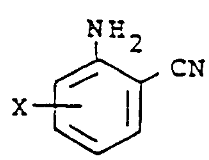

$a_1$) mit einer Verbindung der Formel

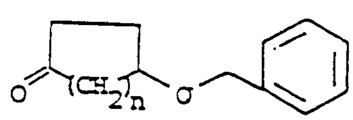

,

worin n den Wert 2 hat, in Anwesenheit einer Lewis-Säure zur Ausbildung einer Verbindung der Formel I'

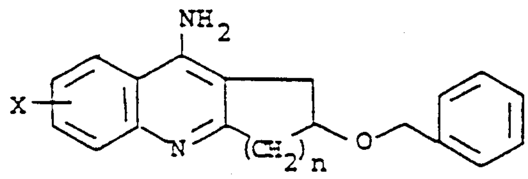

I' ,

worin X und n wie vorstehend definiert sind, oder

$a_2$) mit einer Verbindung der Formel

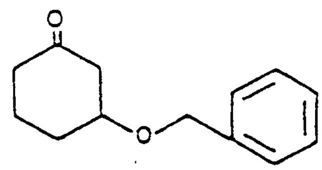

in Anwesenheit einer Lewis-Säure zur Ausbildung einer Verbindung der Formel I"

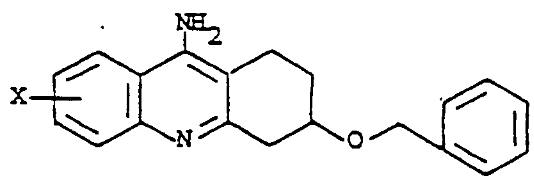

I" ,

worin X die angegebene Definition aufweist, oder

b) Umsetzen einer Verbindung der Formel XVIII

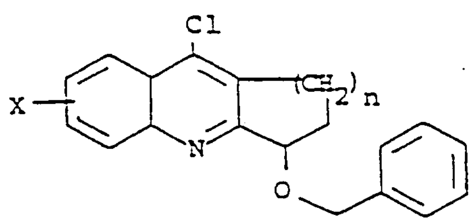

XVIII ,

worin X und n die angegebene Definition aufweisen, mit Ammoniak in einem geeigneten Medium zur Ausbildung einer Verbindung der Formel I"'

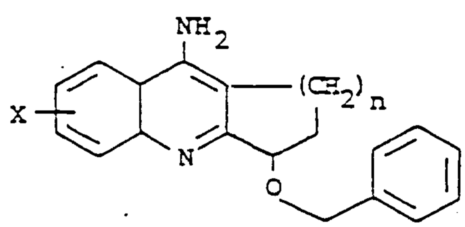

I"' ,

c) gegebenenfalls Umsetzen einer Verbindung der obigen Formeln I', I" oder I"' mit einer Verbindung der Formel $R_1W$, worin W fur Cl, Br, I oder $-OSO_3CH_3$ steht und $R_1$ die obige Definition aufweist, zur Ausbildung einer Verbindung der Formel I, worin R Benzyl bedeutet, $R_1$ wie oben definiert ist, X wie oben definiert ist und n den Wert 2 hat,

d) gegebenenfalls Umsetzen einer Verbindung der Formel I, worin R für Benzyl steht, $R_1$ die obige Definition aufweist, $R_2$ Wasserstoff darstellt, X wie oben definiert ist und n den Wert 2 hat, mit einer Verbindung der Formel $R_2W$, worin W wie in Stufe c) definiert ist und $R_2$ wie oben definiert ist, zur Ausbildung einer Verbindung der Formel I, worin R Benzyl bedeutet und $R_1$, $R_2$, X und n wie

definiert sind,

e) gegebenenfalls Vornahme einer Hydrogenolyse an einer Verbindung der Formel I, worin die Gruppe $-OR$ in 2- oder 3-Stellung vorliegt und R Benzyl bedeutet, $R_1$ und $R_2$ für Wasserstoff, $C_1-C_6$-Alkyl oder Phenyl-$C_1-C_6$-alkyl stehen, worin das Phenyl gegebenenfalls durch 1, 2 oder 3 Substituenten substituiert ist, die jeweils unabhängig voneinander $C_1-C_6$-Alkyl, $C_1-C_6$-Alkoxy, Halogen, Hydroxy oder Trifluormethyl bedeuten, und X und n wie vorstehend definiert sind, unter Ausbildung einer Verbindung der Formel I, worin R für Wasserstoff steht, oder

f) gewünschtenfalls Umsetzen einer Verbindung der Formel I, worin R Wasserstoff bedeutet, $R_1$, $R_2$, X und n wie oben definiert sind, mit einem Acylchlorid der Formel $R_5COCl$ oder mit einem Ester der Formel

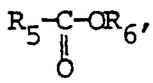

worin $R_5$ für $C_1-C_{11}$-Alkyl steht und $R_6$ für $C_1-C_2$-Alkyl steht, unter Ausbildung einer Verbindung der Formel I, worin R für $C_2-C_{12}$-Alkanoyl steht und $R_1$, $R_2$, X und n wie vorstehend definiert sind, und

g) gewünschtenfalls Umsetzen einer Verbindung der Formel I, worin R für Wasserstoff steht, $R_1$ und $R_2$ Wasserstoff, $C_1-C_6$-Alkyl oder Phenyl-$C_1-C_6$-alkyl bedeuten, worin das Phenyl gewünschtenfalls durch 1, 2 oder 3 Substituenten substituiert ist, die jeweils unabhängig voneinander $C_1-C_6$-Alkyl, $C_1-C_6$-Alkoxy, Halogen, Hydroxy oder Trifluormethyl sind, und X und n wie vorstehend definiert sind, mit einem Formamidacetal der Formel VIII

$R_3R_4NCH(OR_7)_2$ VIII,

worin $R_3$, $R_4$ und $R_7$ für $C_1-C_6$-Alkyl stehen, unter Ausbildung einer Verbindung der Formel I, worin $R_1$ und $R_2$ zusammen die Gruppe $=CH-NR_3R_4$ ausbilden, worin $R_3$ und $R_4$ wie vorstehend definiert sind.

**2.** Verfahren nach Anspruch 1, worin n den Wert 2 hat und die Gruppe $-OR$ in der 2-Stellung vorliegt.

**3.** Verfahren nach Anspruch 2, worin $R_1$, $R_2$ und X Wasserstoff bedeuten.

**4.** Verfahren nach Anspruch 3, worin R für Wasserstoff oder $C_2-C_6$-Alkanoyl steht.

**5.** Verfahren nach Anspruch 1, worin 9-Amino-1,2,3,4-tetrahydroacridin-2-ol oder ein pharmazeutisch annehmbares Säureadditionssalz hievon hergestellt wird.

**6.** Verfahren nach Anspruch 1, worin 2-Acetoxy-1,2,3,4-tetrahydro-9-acridinaminoder ein pharmazeutisch annehmbares Säureadditionssalz hievon hergestellt wird.

**7.** Verfahren nach Anspruch 1, worin 2-Butyryloxy-1,2,3,4-tetrahydro-9-acridinamin oder ein pharmazeutisch annehmbares Säureadditionssalz hievon hergestellt wird.

**8.** Verfahren nach Anspruch 1, worin 2-Hexanoyloxy-1,2,3,4-tetrahydro-9-acridinamin oder ein pharmazeutisch annehmbares Säureadditionssalz hievon hergestellt wird.

**9.** Verwendung einer Verbindung, wie in Anspruch 1 definiert, zur Herstellung eines Medikaments mit einer die Gedächtnisdysfunktion mildernden Aktivität.

**Revendications**

**Revendications pour les Etats contractants suivants : AT, BE, CH, DE, FR, GB, IT, LI, LU, NL, SE**

**1.** Composé de formule I

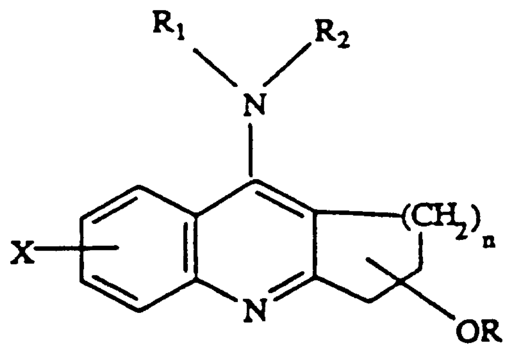

(I)

dans laquelle

| | |
|---|---|
| n | est 2; |
| X | est un atome d'hydrogène ou d'halogène; |
| R | est un atome d'hydrogène ou un groupe benzyle ou alcanoyle ayant de 2 à 12 atomes de carbone, étant entendu que le groupe OR ne peut pas être en position 1 du système tricyclique, ou, si R est un atome d'hydrogène, le groupe OR ne peut pas être en position 4 du système tricyclique; |
| $R_1$ et $R_2$ | sont chacun indépendamment un atome d'hydrogène ou un groupe alkyle en $C_1-C_6$ ou phényl-alkyle($C_1-C_6$), le fragment phényle étant éventuellement substitué par 1, 2 ou 3 substituants qui sont chacun, indépendamment, un atome d'halogène ou un radical alkyle en $C_1-C_6$, alcoxy en $C_1-C_6$, hydroxy ou trifluorométhyle, mais $R_1$ et $R_2$ ne peuvent pas être simultanément l'un et l'autre un groupe phényl-alkyle($C_1-C_6$), ou bien $R_1$ et $R_2$ peuvent former ensemble un groupe |

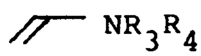

dans lequel $R_3$ et $R_4$ sont chacun indépendamment un groupe alkyle en $C_1-C_6$;

ou un stéréoisomère ou sel d'addition avec un acide pharmaceutiquement acceptable de celui-ci.

**2.** Composé tel que défini dans la revendication 1, dans lequel le groupe OR est en position 2.

**3.** Composé tel que défini dans la revendication 2, dans lequel $R_1$, $R_2$ et X sont des atomes d'hydrogène.

**4.** Composé tel que défini dans la revendication 3, dans lequel R est un atome d'hydrogène ou un groupe alcanoyle en $C_2-C_6$.

**5.** Composé tel que défini dans la revendication 1, qui est le 9-amino-1,2,3,4-tétrahydroacridine-2-ol ou un sel d'addition avec un acide pharmaceutiquement acceptable de celui-ci.

**6.** Composé tel que défini dans la revendication 1, qui est la 2-acétoxy-1,2,3,4-tétrahydro-9-acridinamine ou un sel d'addition avec un acide pharmaceutiquement acceptable de celle-ci.

**7.** Composé tel que défini dans la revendication 1, qui est la 2-butyryloxy-1,2,3,4-tétrahydro-9-acridinamine ou un sel d'addition avec un acide pharmaceutiquement acceptable de celle-ci.

**8.** Composé tel que défini dans la revendication 1, qui est la 2-hexanoyloxy-1,2,3,4-tétrahydro-9-acridinamine ou un sel d'addition avec un acide pharmaceutiquement acceptable de celle-ci.

9. Composition pharmaceutique comprenant, en tant que composant actif, un composé tel que défini dans la revendication 1 et un véhicule approprié pour celui-ci.

10. Utilisation d'un composé tel que défini dans la revendication 1, pour la fabrication d'un médicament ayant une activité atténuant un dysfonctionnement de la mémoire.

11. Procédé pour la préparation d'un composé tel que défini dans la revendication 1, comprenant
a) la mise en réaction d'un composé de formule

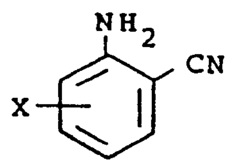

$a_1$) avec un composé de formule

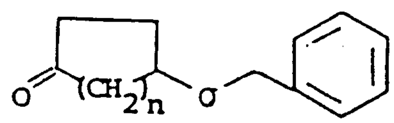

dans laquelle n est 2, en présence d'un acide de Lewis, pour l'obtention d'un composé de formule I'

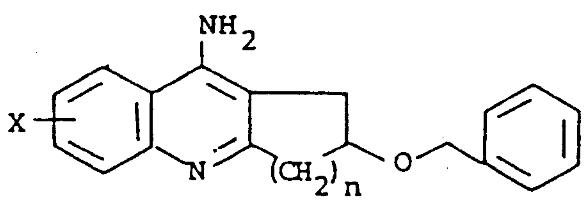

I'

dans laquelle X et n sont tels que définis, ou
$a_2$) avec un composé de formule

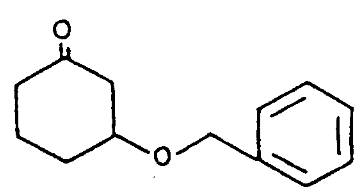

en présence d'un acide de Lewis, pour l'obtention d'un composé de formule I"

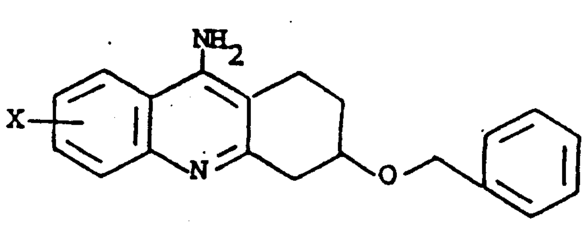

I"

dans laquelle X est tel que défini, ou

b) la mise en réaction d'un composé de formule XVIII

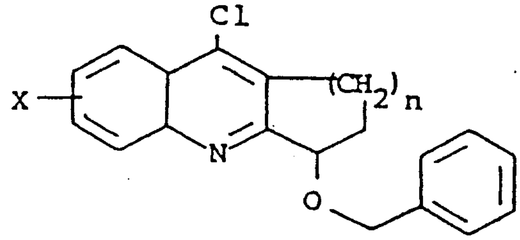

XVIII

dans laquelle X et n sont tels que définis,
avec de l'ammoniac dans un milieu approprié, pour l'obtention d'un composé de formule I'"

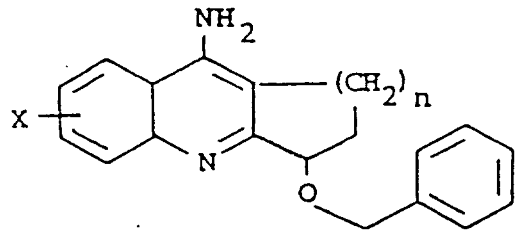

I'"

c) éventuellement la mise en réaction d'un composé de formule I', I" ou I'" ci-dessus avec un composé de formule $R_1W$, W étant Cl, Br, I ou $-OSO_3CH_3$ et $R_1$ étant tel que défini ci-dessus, pour l'obtention d'un composé de formule I dans lequel R est le groupe benzyle, $R_1$ est tel que défini ci-dessus, X est tel que défini ci-dessus et n est 2,
d) éventuellement la mise en réaction d'un composé de formule I dans lequel R est le groupe benzyle, $R_1$ est tel que défini ci-dessus, $R_2$ est un atome d'hydrogène, X est tel que défini ci-dessus et n est 2, avec un composé de formule $R_2W$, W étant tel que défini dans l'étape c) et $R_2$ étant tel que défini ci-dessus, pour l'obtention d'un composé de formule I dans lequel R est le groupe benzyle et $R_1$, $R_2$, X et n sont tels que définis,
e) éventuellement la soumission d'un composé de formule I dans lequel le groupe OR est en position 2 ou 3, et R est le groupe benzyle, $R_1$ et $R_2$ sont un atome d'hydrogène ou un groupe alkyle en $C_1-C_6$ ou phényl-alkyle($C_1-C_6$), le fragment phényle étant éventuellement substitué par 1, 2 ou 3 substituants qui sont chacun indépendamment un atome d'halogène ou un groupe alkyle en $C_1-C_6$, alcoxy en $C_1-C_6$, hydroxy ou trifluorométhyle, X et n sont tels que définis ci-dessus, à une hydrogénolyse pour l'obtention d'un composé de formule I dans lequel R est un atome d'hydrogène, ou
f) éventuellement la mise en réaction d'un composé de formule I, dans lequel R est un atome d'hydrogène, $R_1$, $R_2$, X et n sont tels que définis ci-dessus, avec un chlorure d'acyle de formule $R_5COCl$ ou un ester de formule

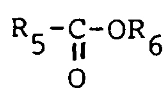

$R_5$ étant un groupe alkyle en $C_1-C_{11}$ et $R_6$ étant un groupe alkyle en $C_1-C_2$, pour l'obtention d'un composé de formule I dans lequel R est un groupe alcanoyle en $C_2-C_{12}$ et $R_1$, $R_2$, X et n sont tels que définis ci-dessus, et
g) éventuellement la mise en réaction d'un composé de formule I, dans lequel R est un atome d'hydrogène, $R_1$ et $R_2$ sont un atome d'hydrogène ou un groupe alkyle en $C_1-C_6$ ou phényl-alkyle($C_1-C_6$), le fragment phényle étant éventuellement substitué par 1, 2 ou 3 substituants qui sont chacun indépendamment un atome d'halogène ou un groupe alkyle en $C_1-C_6$, alcoxy en $C_1-C_6$, hydroxy ou trifluorométhyle, et X et n sont tels que définis, avec un formamide-acétal de formule VIII

$R_3R_4NCH(OR_7)_2$ VIII

dans laquelle $R_3$, $R_4$ et $R_7$ sont des groupes alkyle en $C_1-C_6$, pour l'obtention d'un composé de formule I dans lequel $R_1$ et $R_2$ forment ensemble le groupe $=CH-NR_3R_4$, $R_3$ et $R_4$ étant tels que définis.

**Revendications pour les Etats contractants suivants : ES, GR**

**1.** Procédé pour la préparation d'un composé de formule I

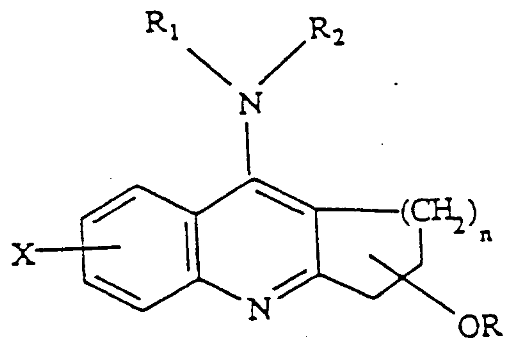

(I)

dans laquelle

| | |
|---|---|
| n | est 2, |
| X | est un atome d'hydrogène ou d'halogène, |
| R | est un atome d'hydrogène ou un groupe benzyle ou alcanoyle ayant de 2 à 12 atomes de carbone, étant entendu que le groupe OR ne peut pas être en position 1 du système tricyclique, ou, si R est un atome d'hydrogène, le groupe OR ne peut pas être en position 4 du système tricyclique, |
| $R_1$ et $R_2$ | sont chacun indépendamment un atome d'hydrogène ou un groupe alkyle en $C_1-C_6$ ou phényl-alkyle($C_1-C_6$), le fragment phényle étant éventuellement substitué par 1, 2 ou 3 substituants qui sont chacun, indépendamment, un atome d'halogène ou un radical alkyle en $C_1-C_6$, alcoxy en $C_1-C_6$, hydroxy ou trifluorométhyle, mais $R_1$ et $R_2$ ne peuvent pas être simultanément l'un et l'autre un groupe phényl-alkyle($C_1-C_6$), ou bien $R_1$ et $R_2$ peuvent former ensemble un groupe |

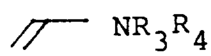

dans lequel $R_3$ et $R_4$ sont chacun indépendamment un groupe alkyle inférieur,

ou d'un stéréoisomère ou d'un sel d'addition avec un acide pharmaceutiquement acceptable de celui-ci,

comprenant

a) la mise en réaction d'un composé de formule

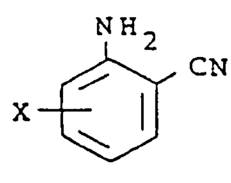

$a_1$) avec un composé de formule

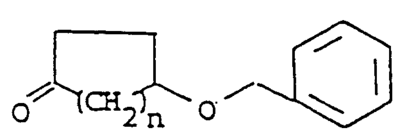

dans laquelle n est 2, en présence d'un acide de Lewis, pour l'obtention d'un composé de formule I'

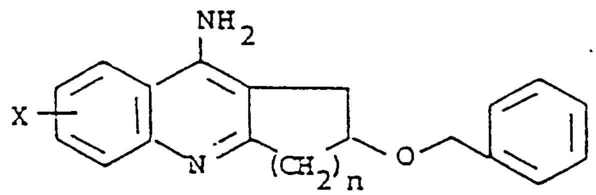

I'

dans laquelle X et n sont tels que définis, ou

$a_2$) avec un composé de formule

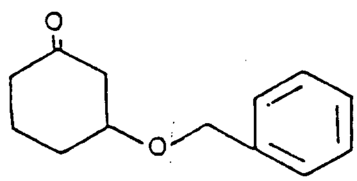

en présence d'un acide de Lewis, pour l'obtention d'un composé de formule I"

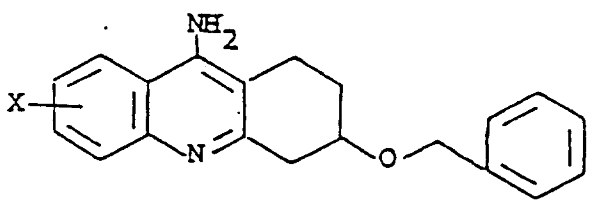

I"

dans laquelle X est tel que défini, ou

b) la mise en réaction d'un composé de formule XVIII

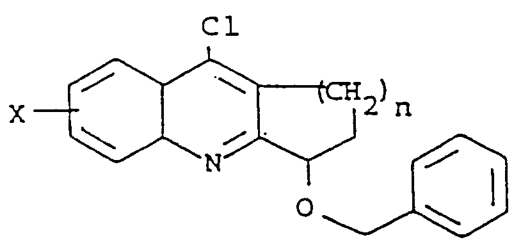

XVIII

dans laquelle X et n sont tels que définis,

avec de l'ammoniac dans un milieu approprié, pour l'obtention d'un composé de formule I'"

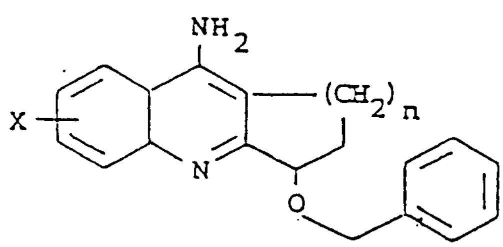

I'"

c) éventuellement la mise en réaction d'un composé de formule I', I" ou I'" ci-dessus avec un composé de formule $R_1W$, W étant Cl, Br, I ou $-OSO_3CH_3$ et $R_1$ étant tel que défini ci-dessus,

pour l'obtention d'un composé de formule I dans lequel R est le groupe benzyle, $R_1$ est tel que défini ci-dessus, X est tel que défini ci-dessus et n est 2,

d) éventuellement la mise en réaction d'un composé de formule I dans lequel R est le groupe benzyle, $R_1$ est tel que défini ci-dessus, $R_2$ est un atome d'hydrogène, X est tel que défini et n est 2, avec un composé de formule $R_2W$, W étant tel que défini dans l'étape c) et $R_2$ étant tel que défini ci-dessus, pour l'obtention d'un composé de formule I dans lequel R est le groupe benzyle et $R_1$, $R_2$, X et n sont tels que définis,

e) éventuellement la soumission d'un composé de formule I dans lequel le groupe OR est en position 2 ou 3, et R est le groupe benzyle, $R_1$ et $R_2$ sont un atome d'hydrogène ou un groupe alkyle en $C_1-C_6$ ou phényl-alkyle($C_1-C_6$), le fragment phényle étant éventuellement substitué par 1, 2 ou 3 substituants qui sont chacun indépendamment un atome d'halogène ou un groupe alkyle en $C_1-C_6$, alcoxy en $C_1-C_6$, hydroxy ou trifluorométhyle, X et n sont tels que définis ci-dessus, à une hydrogénolyse pour l'obtention d'un composé de formule I dans lequel R est un atome d'hydrogène, ou

f) éventuellement la mise en réaction d'un composé de formule I, dans lequel R est un atome d'hydrogène, $R_1$, $R_2$, X et n sont tels que définis ci-dessus, avec un chlorure d'acyle de formule $R_5COCl$ ou un ester de formule

$$R_5-\underset{\underset{O}{\|}}{C}-OR_6$$

$R_5$ étant un groupe alkyle en $C_1-C_{11}$ et $R_6$ étant un groupe alkyle en $C_1-C_2$, pour l'obtention d'un composé de formule I dans lequel R est un groupe alcanoyle en $C_2-C_{12}$ et $R_1$, $R_2$, X et n sont tels que définis ci-dessus, et

g) éventuellement la mise en réaction d'un composé de formule I, dans lequel R est un atome d'hydrogène, $R_1$ et $R_2$ sont un atome d'hydrogène ou un groupe alkyle en $C_1-C_6$ ou phényl-alkyle($C_1-C_6$), le fragment phényle étant éventuellement substitué par 1, 2 ou 3 substituants qui sont chacun indépendamment un atome d'halogène ou un groupe alkyle en $C_1-C_6$, alcoxy en $C_1-C_6$, hydroxy ou trifluorométhyle, et X et n sont tels que définis, avec un formamide-acétal de formule VIII

$R_3R_4NCH(OR_7)_2$ VIII

dans laquelle $R_3$, $R_4$ et $R_7$ sont des groupes alkyle en $C_1-C_6$, pour l'obtention d'un composé de formule I dans lequel $R_1$ et $R_2$ forment ensemble le groupe $=CH-NR_3R_4$, $R_3$ et $R_4$ étant tels que définis.

**2.** Procédé selon la revendication 1, dans lequel n est 2 et le groupe OR est en position 2.

**3.** Procédé selon la revendication 2, dans lequel $R_1$, $R_2$ et X sont des atomes d'hydrogène.

**4.** Procédé selon la revendication 3, dans lequel R est un atome d'hydrogène ou un groupe alcanoyle en $C_2-C_6$.

**5.** Procédé selon la revendication 1, dans lequel on prépare le 9-amino-1,2,3,4-tétrahydroacridine-2-ol ou un sel d'addition avec un acide pharmaceutiquement acceptable de celui-ci.

**6.** Procédé selon la revendication 1, dans lequel on prépare la 2-acétoxy-1,2,3,4-tétrahydro-9-acridinamine ou un sel d'addition avec un acide pharmaceutiquement acceptable de celle-ci.

**7.** Procédé selon la revendication 1, dans lequel on prépare la 2-butyryloxy-1,2,3,4-tétrahydro-9-acridinamine ou un sel d'addition avec un acide pharmaceutiquement acceptable de celle-ci.

**8.** Procédé selon la revendication 1, dans lequel on prépare la 2-hexanoyloxy-1,2,3,4-tétrahydro-9-acridinamine ou un sel d'addition avec un acide pharmaceutiquement acceptable de celle-ci.

9. Utilisation d'un composé tel que défini dans la revendication 1, pour la fabrication d'un médicament ayant une activité atténuant un dysfonctionnement de la mémoire.